(19) Europäisches Patentamt
European Patent Office
Office européen des brevets

(11) Publication number : **0 518 636 A1**

(12) # EUROPEAN PATENT APPLICATION

(21) Application number : **92305315.1**

(22) Date of filing : **10.06.92**

(51) Int. Cl.$^5$ : **C09K 19/32,** C09K 19/34, C09K 19/42, C07C 43/225, C07C 43/215, C07C 69/76, C07C 69/94, C07C 43/20, C07C 43/205, C07D 213/06, C07D 213/30, C07D 213/55, C07D 213/64, C07D 213/79

(30) Priority : **11.06.91 JP 167604/91**
**14.06.91 JP 170617/91**
**27.06.91 JP 183713/91**

(43) Date of publication of application :
**16.12.92 Bulletin 92/51**

(84) Designated Contracting States :
**DE FR GB IT**

(71) Applicant : **SANYO CHEMICAL INDUSTRIES LTD.**
**11-1, Ichinohashinomoto-cho Higashiyama-ku, Kyoto-shi**
**Kyoto 605 (JP)**

(72) Inventor : **Satoh, Masahiro**
**Corporouse Fushimi 112, No 32**
**Fukakusa-ikenouchi-c**
**Fushimi-ku, Kyoto (JP)**
Inventor : **Sugita, Naoko**
**No 12-44 Higashinominamiinoue-cho**
**Yamashina-ku, Kyoto (JP)**
Inventor : **Watanabe, Tetsuya**
**No 71-38 Muranohigashi-machi**
**Hirakata-shi, Osaka-fu (JP)**
Inventor : **Yoshio, Kunikiyo**
**No 1922-361 Noji-cho**
**Kusatsu, Shiga-ken (JP)**
Inventor : **Yanagi, Tatsuroh**
**No 10-1, Imakumano-minamihiyoshi-cho**
**Higashiyama-ku, Kyoto (JP)**

(74) Representative : **Marlow, Nicholas Simon**
**Reddie & Grose 16, Theobalds Road**
**London WC1X 8PL (GB)**

(54) **Liquid crystal compounds and compositions.**

(57) Liquid crystal naphthalene compounds, represented by the following formula (1) are disclosed.

R-Z-A-&num ;-NAP-Z′-R′ (1)

In the formula (1), R is an alkyl group containing 1 to 18 carbon atoms ; R′ is an alkyl group containing 1 to 21 carbon atoms ; NAP represents 2,6-naphthylene group ;&num ; represents C≡C ;
A, Z and Z′ are as follows : 1) A is Pyr> and
(a) Z is - and Z′ is O or COO or (b) Z is OCO and Z′ is - or O ; 2) A is FPhF and (a) Z is - or O and Z′ is -, O or COO or (b) Z is OCO and Z′ is - or O ; 3) A is FPh and (a) Z is - and Z′ is O or COO, (b) Z is O and Z′ is -, O or COO or (c) Z is OCO and Z′ is - or O ; 4) A is <Pyr and Z is - or O and Z′ is COO ; or 5) A is PhF and Z is O and Z′ is - ; wherein :

Pyr> = 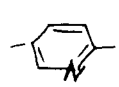, FPhF = 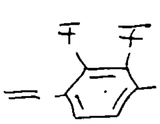, FPh = 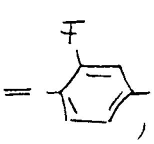,
<Pyr = 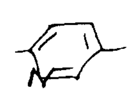, PhF = 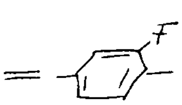 and – represents a single bond.

When used as a component of liquid crystal composition showing ciral smectic C phase, these compounds can provide liquid crystal compositions of improved orientation and free from zigzag deffects, and liquid crystal display elements of high contrast ratio.

EP 0 518 636 A1

## BACKGROUND OF THE INVENTION

### 1. Field of the Invention

This invention relates to liquid crystal compounds and liquid crystal compositions, useful for liquid crystal display elements and the like. More particularly, it relates to liquid crystal naphthalene compounds showing non-chiral smectic C phase (hereinafter referred to as Sc phase) or chiral smectic C phase (hereinafter referred to as Sc∗ phase) and liquid crystal compositions containing these liquid crystal compounds.

### 2. Description of the Prior Art

As liquid crystal compounds showing Sc phase or Sc∗ phase, used in ferroelectric liquid crystal materials, there have been known liquid crystal phenyl pyrimidine compounds, such as 5-alkyl-2-(4′-alkoxyphenyl)pyrimidines.

However, these liquid crystal compounds and compositions containing these compounds are likely result in zigzag deffects, because of poor orientation, when introduced into cells, and cannot provide sufficiently high contrast ratio.

The present invention can provide a liquid crystal compound showing Sc or Sc∗ phase of improved orientation.

The present invention can provide a liquid crystal compound capable of providing ferroelectric liquid crystal composition substantially free from zigzag deffects.

The present invention can further provide a liquid crystal compound and composition capable of providing liquid crystal display elements of improved contrast ratio.

The present invention can still further provide a liquid crystal compound, showing Sc or Sc∗ phase over a wide temperature range.

The present invention provides liquid crystal compounds represented by the following general formula (1):

R-Z-A-#-NAP-Z′-R′     (1)

In the formula (1), R is an alkyl group containing 1 to 18 carbon atoms; R′ is an alkyl group containing 1 to 21 carbon atoms; A is a cyclic group selected from the group consisting of Pyr>, FPhF, FPh, <Pyr and PhF; Z is -, O or OCO, and Z′ is -, O or COO;
with the provisos that

1) if A is Pyr>,
   (a) Z is - and Z′ is O or COO, or
   (b) Z is OCO and Z′ is - or O;
2) if A is FPhF,
   (a) Z is - or O and Z′ is -, O or COO, or
   (b) Z is OCO and Z′ is - or O;
3) if A is FPh,
   (a) Z is - and Z′ is O or COO,
   (b) Z is O and Z′ is -, O or COO, or
   (c) Z is OCO and Z′ is - or O;
4) if A is <Pyr, Z is - or O and Z′ is COO; and
5) if A is PhF, Z is O and Z′ is -.

In the above and hereinafter, NAP, #, Pyr>, FPhF, FPh, <Pyr, PhF and - represent, respectively, 2,6-naphthylene group, C≡C (C-C triple bond),

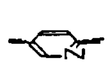 , 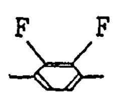 , 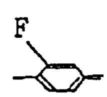 ,

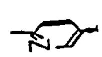 , 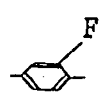 ,

and a single bond (direct bond).

DETAILED DESCRIPTION OF THE PREFERRED EMBODIMENTS

In the general formula (1), R and R′ are the same or different alkyl groups containing usually 1 - 18 carbon atoms, which may be substituted with at least one substituent selected from the group consisting of fluorine, chlorine, alkoxy groups containing 1 to 10 or more carbon atoms, trifluoromethyl group and cyano group.

Illustrative of suitable alkyl groups are straight-chain alkyl groups, branched alkyl groups, cyclic alkyl groups, F-substituted alkyl groups, Cl-substituted alkyl groups and alkoxy-substituted alkyl groups, as written in EP 0 385 692 as examples of R and R′; trifluoromethyl-substituted alkyl groups, such as 1-trifluoromethylpropyl, 1-trifluoromethylbutyl, 1-trifluoromethylpentyl, 1-trifluoromethylhexyl, 1-trifluoromethylheptyl, 1-trifluoromethyloctyl, 1-trifluoromethylnonyl, 1-trifluoromethyldecyl, 1-trifluoromethylundecyl, 1-trifluoromethyldodecyl, 1-trifluoromethyl-2-methoxyethyl, 1-trifluoromethyl-2-ethoxyethyl, 1-trifluoromethyl-2-propoxyethyl, 1-trifluoromethyl-2-butoxyethyl, 1-trifluoromethyl-2-pentyloxyethyl, 1-trifluoromethyl-2-hexyloxyethyl, 1-trifluoromethyl-2-isopropyloxyethyl, 1-trifluoromethyl-2-(1′-methylpropyloxy)ethyl, 1-trifluoromethyl-2-(1′-methylbutyloxy)ethyl, 1-trifluoromethyl -2-(1′-methylpentyloxy)ethyl, 1-trifluoromethyl-2-(1′-methylhexyloxy)ethyl, 1-trifluoromethyl-2-(1′-methylheptyloxy)ethyl, 1-trifluoromethyl-2-(2′-methylbutyloxy)ethyl, 1-trifluoromethyl-3-methoxypropyl, 1-trifluoromethyl-3-ethoxypropyl, 1-trifluoromethyl-3-propoxypropyl, 1-trifluoromethyl-3-butyloxypropyl, 1-trifluoromethyl-3-pentyloxypropyl, 1-trifluoromethyl-3-hexyloxypropyl, 1-trifluoromethyl -3-iso-propyloxypropyl, 1-trifluoromethyl-3-(1′-methylpropyloxy)propyl, 1-trifluoromethyl-3-(1′-methylbutyloxy)propyl, 1-trifluoromethyl-3-(1′-methylpentyloxy)propyl, 1-trifluoromethyl-3-(1′-methylhexyloxy)propyl, 1-trifluoromethyl -3-(1′-methylheptyloxy)propyl, 1-trifluoromethyl-3-(2′-methylbutyloxy)propyl, 1-trifluoromethyl-4-methoxybutyl; 1-trifluoromethyl-4-ethoxybutyl, 1-trifluoromethyl-4-propyloxybutyl, 1-trifluoromethyl-4-butyloxybutyl, 1-trifluoromethyl-4-pentyloxybutyl, 1-trifluoromethyl-4-hexyloxybutyl, 1-trifluoromethyl-4-iso-propyloxybutyl, 1-trifluoromethyl-4-(1′-methylpropyloxy)butyl, 1-trifluoromethyl-4-(1′-methylbutyloxy)butyl, 1-trifluoromethyl-4-(1′-methylpentyloxy)butyl, 1-trifluoromethyl-4-(1′-methylhexyloxy)butyl, 1-trifluoromethyl-4-(1′-methylheptyloxy)butyl, and 1-trifluoromethyl-4-(2′-methylbutyloxy)butyl; fluoro-substituted alkyl groups, such as 2-fluoropropyl, 2-fluorobutyl, 2-fluoropentyl, 2-fluorohexyl, 2-fluoroheptyl, 2-fluorooctyl, 2-fluorononyl, 2-fluorodecyl, 2-fluoroundecyl and 2-fluorododecyl; cyano-substituted alkyl groups, such as 1-cyanopropyl, 1-cyanobutyl, 1-cyanopentyl, 1-cyanohexyl, 1-cyanoheptyl, 1-cyanooctyl, 1-cyanononyl, 1-cyanodecyl, 1-cyanoundecyl and 1-cyanododecyl; and unsaturated alkyl groups (alkenyl groups), such as 2-propenyl, 3-butenyl, 4-pentenyl, 5-hexenyl, 6-heptenyl, 7-octenyl, 8-nonenyl, 9-decenyl, 11-dodecenyl, 17-octadecenyl, trans-2-decenyl, trans-5-decenyl, cis-3-nonenyl, cis-6-nonenyl, trans-2-heptenyl, cis-2-hexenyl, trans-2-hexenyl, cis-3-hexenyl, cis-4-hexenyl, and the like.

Alkyl groups R and R′ may contain one or more asymmetric carbon atoms, and may be optically active (hereinafter referred to as oa). Illustrative of suitable oa alkyl groups are oa alkyl groups, F-substituted oa alkyl groups, Cl-substituted oa alkyl groups and alkoxy-substituted oa alkyl groups, as written in EP 0 385 692 as examples of R and R′; as well as oa ones among trifluoromethyl-substituted alkyl groups and cyano-substituted as mentioned hereinabove. Alkyl groups of R′ may be of the formula: $-(CH_2)_nC{*}H(CH_3)R''$, wherein R″ is an alkyl group containing 2-10 (particularly 2-6) carbon atoms and n is an integer of 0-9 (particularly 0-5).

Among these, preferred are alkyl groups containing 1 to 16, particularly 1 to 14 carbon atoms.

Illustrative examples of the compounds represented by the formula (1) include those shown in Table 1 to Table 11. In these Tables and also hereinafter, Me, Et, PRO, BUT, PEN, HEX, HEP, OCT, NON, DEC, 2MB, 4MH, 1MH, 2FO, HEXE, HEPE, OCTE, NONE and DECE represent methyl, ethyl, n-propyl, n-butyl, n-pentyl, n-hexyl, n-heptyl, n-octyl, n-nonyl, n-decyl, 2-methylbutyl[$-CH_2{*}CH(Me)Et$], 4-methylhexyl[$-(CH_2)_3{*}CH(Me)Et$], 1-methylheptyl[$-{*}CH(Me)HEX$], 2-fluorooctyl[$-CH_2{*}CHF-HEX$], 5-hexenyl, 6-heptenyl, 7-octenyl, 8-nonenyl and 9-decenyl, respectively.

Table 1. Formula (1) wherein Z is - and z' is 0

| No | R | A | R' | No | R | A | R' | No | R | A | R' |
|---|---|---|---|---|---|---|---|---|---|---|---|
| 1 | HEP | Pyr> | OCT | 26 | NON | FPhF | NON | 51 | OCT | FPhF | NONE |
| 2 | HEP | Pyr> | NON | 27 | DEC | FPhF | HEX | 52 | OCT | FPhF | DECE |
| 3 | HEP | Pyr> | DEC | 28 | DEC | FPhF | HEP | 53 | NON | FPhF | HEXE |
| 4 | OCT | Pyr> | HEP | 29 | DEC | FPhF | OCT | 54 | NON | FPhF | HEPE |
| 5 | OCT | Pyr> | OCT | 30 | DEC | FPhF | NON | 55 | NON | FPhF | OCTE |
| 6 | OCT | Pyr> | NON | 31 | HEP | Pyr> | OCTE | 56 | NON | FPhF | NONE |
| 7 | OCT | Pyr> | DEC | 32 | HEP | Pyr> | NONE | 57 | DEC | FPhF | HEXE |
| 8 | NON | Pyr> | HEX | 33 | HEP | Pyr> | DECE | 58 | DEC | FPhF | HEPE |
| 9 | NON | Pyr> | HEP | 34 | OCT | Pyr> | HEPE | 59 | DEC | FPhF | OCTE |
| 10 | NON | Pyr> | OCT | 35 | OCT | Pyr> | OCTE | 60 | DEC | FPhF | NONE |
| 11 | NON | Pyr> | NON | 36 | OCT | Pyr> | NONE | 61 | DEC | Pyr> | 4MH |
| 12 | DEC | Pyr> | HEX | 37 | OCT | Pyr> | DECE | 62 | DEC | Pyr> | 2MB |
| 13 | DEC | Pyr> | HEP | 38 | NON | Pyr> | HEXE | 63 | DEC | Pyr> | 1MH |
| 14 | DEC | Pyr> | OCT | 39 | NON | Pyr> | HEPE | 64 | DEC | Pyr> | 2FO |
| 15 | DEC | Pyr> | NON | 40 | NON | Pyr> | OCTE | 65 | DEC | FPhF | 4MH |
| 16 | HEP | FPhF | OCT | 41 | NON | Pyr> | NONE | 66 | DEC | FPhF | 2MB |
| 17 | HEP | FPhF | NON | 42 | DEC | Pyr> | HEXE | 67 | NON | FPhF | 1MH |
| 18 | HEP | FPhF | DEC | 43 | DEC | Pyr> | HEPE | 68 | DEC | FPhF | 2FO |
| 19 | OCT | FPhF | HEP | 44 | DEC | Pyr> | OCTE | 69 | DEC | FPh | OCT |
| 20 | OCT | FPhF | OCT | 45 | DEC | Pyr> | NONE | | | | |
| 21 | OCT | FPhF | NON | 46 | HEP | FPhF | OCTE | | | | |
| 22 | OCT | FPhF | DEC | 47 | HEP | FPhF | NONE | | | | |
| 23 | NON | FPhF | HEX | 48 | HEP | FPhF | DECE | | | | |
| 24 | NON | FPhF | HEP | 49 | OCT | FPhF | HEPE | | | | |
| 25 | NON | FPhF | OCT | 50 | OCT | FPhF | OCTE | | | | |

Table 2. Formula (1) wherein Z is O and z' is -

| No | R | A | R' | No | R | A | R' | No | R | A | R' |
|---|---|---|---|---|---|---|---|---|---|---|---|
| 101 | HEP | FPhF | OCT | 126 | NONE | FPhF | NON | 151 | OCTE | FPh | NON |
| 102 | HEP | FPhF | NON | 127 | DECE | FPhF | HEX | 152 | OCTE | FPh | DEC |
| 103 | HEP | FPhF | DEC | 128 | DECE | FPhF | HEP | 153 | NONE | FPh | HEX |
| 104 | OCT | FPhF | HEP | 129 | DECE | FPhF | OCT | 154 | NONE | FPh | HEP |
| 105 | OCT | FPhF | OCT | 130 | DECE | FPhF | NON | 155 | NONE | FPh | OCT |
| 106 | OCT | FPhF | NON | 131 | HEP | FPh | OCT | 156 | NONE | FPh | NON |
| 107 | OCT | FPhF | DEC | 132 | HEP | FPh | NON | 157 | DECE | FPh | HEX |
| 108 | NON | FPhF | HEX | 133 | HEP | FPh | DEC | 158 | DECE | FPh | HEP |
| 109 | NON | FPhF | HEP | 134 | OCT | FPh | HEP | 159 | DECE | FPh | OCT |
| 110 | NON | FPhF | OCT | 135 | OCT | FPh | OCT | 160 | DECE | FPh | NON |
| 111 | NON | FPhF | NON | 136 | OCT | FPh | NON | 161 | 4MH | FPh | DEC |
| 112 | DEC | FPhF | HEX | 137 | OCT | FPh | DEC | 162 | 2MB | FPh | DEC |
| 113 | DEC | FPhF | HEP | 138 | NON | FPh | HEX | 163 | 1MH | FPh | DEC |
| 114 | DEC | FPhF | OCT | 139 | NON | FPh | HEP | 164 | 2FO | FPh | DEC |
| 115 | DEC | FPhF | NON | 140 | NON | FPh | OCT | 165 | NON | PhF | NON |
| 116 | HEPE | FPhF | OCT | 141 | NON | FPh | NON | 166 | 4MH | FPhF | DEC |
| 117 | HEPE | FPhF | NON | 142 | DEC | FPh | HEX | 167 | 2MB | FPhF | DEC |
| 118 | HEPE | FPhF | DEC | 143 | DEC | FPh | HEP | 168 | 1MH | FPhF | DEC |
| 119 | OCTE | FPhF | HEP | 144 | DEC | FPh | OCT | 169 | 2FO | FPhF | DEC |
| 120 | OCTE | FPhF | OCT | 145 | DEC | FPh | NON | | | | |
| 121 | OCTE | FPhF | NON | 146 | HEPE | FPh | OCT | | | | |
| 122 | OCTE | FPhF | DEC | 147 | HEPE | FPh | NON | | | | |
| 123 | NONE | FPhF | HEX | 148 | HEPE | FPh | DEC | | | | |
| 124 | NONE | FPhF | HEP | 149 | OCTE | FPh | HEP | | | | |
| 125 | NONE | FPhF | OCT | 150 | OCTE | FPh | OCT | | | | |

Table 3. Formula (1) wherein Z is 0 and z' is 0

| No | R | A | R' | No | R | A | R' | No | R | A | R' |
|---|---|---|---|---|---|---|---|---|---|---|---|
| 201 | HEP | FPhF | OCT | 226 | NON | FPhF | NONE | 251 | 2MB | FPhF | DEC |
| 202 | HEP | FPhF | NON | 227 | DEC | FPhF | HEXE | 252 | 1MH | FPhF | DEC |
| 203 | HEP | FPhF | DEC | 228 | DEC | FPhF | HEPE | 253 | 2FO | FPhF | DEC |
| 204 | OCT | FPhF | HEP | 229 | DEC | FPhF | OCTE | 254 | OCT | FPh | OCT |
| 205 | OCT | FPhF | OCT | 230 | DEC | FPhF | NONE | 255 | DEC | FPh | 2MB |
| 206 | OCT | FPhF | NON | 231 | HEPE | FPhF | OCT | 256 | DEC | <Pyr | 2MB |
| 207 | OCT | FPhF | DEC | 232 | HEPE | FPhF | NON | 257 | DEC | <Pyr | OCT |
| 208 | NON | FPhF | HEX | 233 | HEPE | FPhF | DEC | | | | |
| 209 | NON | FPhF | HEP | 234 | OCTE | FPhF | HEP | | | | |
| 210 | NON | FPhF | OCT | 235 | OCTE | FPhF | OCT | | | | |
| 211 | NON | FPhF | NON | 236 | OCTE | FPhF | NON | | | | |
| 212 | DEC | FPhF | HEX | 237 | OCTE | FPhF | DEC | | | | |
| 213 | DEC | FPhF | HEP | 238 | NONE | FPhF | HEX | | | | |
| 214 | DEC | FPhF | OCT | 239 | NONE | FPhF | HEP | | | | |
| 215 | DEC | FPhF | NON | 240 | NONE | FPhF | OCT | | | | |
| 216 | HEP | FPhF | OCTE | 241 | NONE | FPhF | NON | | | | |
| 217 | HEP | FPhF | NONE | 242 | DECE | FPhF | HEX | | | | |
| 218 | HEP | FPhF | DECE | 243 | DECE | FPhF | HEP | | | | |
| 219 | OCT | FPhF | HEPE | 244 | DECE | FPhF | OCT | | | | |
| 220 | OCT | FPhF | OCTE | 245 | DECE | FPhF | NON | | | | |
| 221 | OCT | FPhF | NONE | 246 | DEC | FPhF | 4MH | | | | |
| 222 | OCT | FPhF | DECE | 247 | DEC | FPhF | 2MB | | | | |
| 223 | NON | FPhF | HEXE | 248 | DEC | FPhF | 1MH | | | | |
| 224 | NON | FPhF | HEPE | 249 | DEC | FPhF | 2FO | | | | |
| 225 | NON | FPhF | OCTE | 250 | 4MH | FPhF | OCT | | | | |

Table 4. Formula (1) wherein Z is O and z' is COO

| No | R | A | R' | No | R | A | R' | No | R | A | R' |
|---|---|---|---|---|---|---|---|---|---|---|---|
| 301 | DEC | FPhF | 4MH | 307 | DEC | FPh | 4MH | 313 | DEC | <Pyr | 4MH |
| 302 | DEC | FPhF | 2MB | 308 | DEC | FPh | 2MB | 314 | DEC | <Pyr | 2MB |
| 303 | DEC | FPhF | 1MH | 309 | DEC | FPh | 1MH | 315 | DEC | <Pyr | 1MH |
| 304 | DEC | FPhF | 2FO | 310 | DEC | FPh | 2FO | 316 | DEC | <Pyr | 2FO |
| 305 | DEC | FPhF | OCT | 311 | DEC | FPh | OCT | 317 | DEC | <Pyr | OCT |
| 306 | OCT | FPhF | DEC | 312 | OCT | FPh | DEC | 318 | OCT | <Pyr | DEC |

Table 5. Formula (1) wherein Z is - and z' is COO

| No | R | A | R' | No | R | A | R' | No | R | A | R' |
|---|---|---|---|---|---|---|---|---|---|---|---|
| 401 | DEC | Pyr> | 4MH | 407 | DEC | FPhF | 4MH | 413 | DEC | FPh | 4MH |
| 402 | DEC | Pyr> | 2MB | 408 | DEC | FPhF | 2MB | 414 | DEC | FPh | 2MB |
| 403 | DEC | Pyr> | 1MH | 409 | DEC | FPhF | 1MH | 415 | DEC | FPh | 1MH |
| 404 | DEC | Pyr> | 2FO | 410 | DEC | FPhF | 2FO | 416 | DEC | FPh | 2FO |
| 405 | DEC | Pyr> | OCT | 411 | DEC | FPhF | OCT | 417 | DEC | FPh | OCT |
| 406 | OCT | Pyr> | DEC | 412 | OCT | FPhF | DEC | 418 | OCT | FPh | DEC |

Table 6. Formula (1) wherein Z is OCO and z' is -

| No | R | A | R' | No | R | A | R' | No | R | A | R' |
|---|---|---|---|---|---|---|---|---|---|---|---|
| 501 | 4MH | Pyr> | DEC | 507 | 4MH | FPhF | DEC | 513 | 4MH | FPh | DEC |
| 502 | 2MB | Pyr> | DEC | 508 | 2MB | FPhF | DEC | 514 | 2MB | FPh | DEC |
| 503 | 1MH | Pyr> | DEC | 509 | 1MH | FPhF | DEC | 515 | 1MH | FPh | DEC |
| 504 | 2FO | Pyr> | DEC | 510 | 2FO | FPhF | DEC | 516 | 2FO | FPh | DEC |
| 505 | OCT | Pyr> | DEC | 511 | OCT | FPhF | DEC | 517 | OCT | FPh | DEC |
| 506 | DEC | Pyr> | OCT | 512 | DEC | FPhF | OCT | 518 | DEC | FPh | OCT |

Table 7. Formula (1) wherein Z is OCO and z' is O

| No | R | A | R' | No | R | A | R' | No | R | A | R' |
|---|---|---|---|---|---|---|---|---|---|---|---|
| 601 | 4MH | Pyr> | DEC | 607 | 4MH | FPhF | DEC | 613 | 4MH | FPh | DEC |
| 602 | 2MB | Pyr> | DEC | 608 | 2MB | FPhF | DEC | 614 | 2MB | FPh | DEC |
| 603 | 1MH | Pyr> | DEC | 609 | 1MH | FPhF | DEC | 615 | 1MH | FPh | DEC |
| 604 | 2FO | Pyr> | DEC | 610 | 2FO | FPhF | DEC | 616 | 2FO | FPh | DEC |
| 605 | OCT | Pyr> | DEC | 611 | OCT | FPhF | DEC | 617 | OCT | FPh | DEC |
| 606 | DEC | Pyr> | OCT | 612 | DEC | FPhF | OCT | 618 | DEC | FPh | OCT |

Table 8. Formula (1) wherein Z is OCO and z' is O

| No | R | A | R' | No | R | A | R' | No | R | A | R' |
|---|---|---|---|---|---|---|---|---|---|---|---|
| 701 | HEP | FPhF | OCT | 706 | OCT | FPhF | NON | 711 | NON | FPhF | NON |
| 702 | HEP | FPhF | NON | 707 | OCT | FPhF | DEC | 712 | DEC | FPhF | HEX |
| 703 | HEP | FPhF | DEC | 708 | NON | FPhF | HEX | 713 | DEC | FPhF | HEP |
| 704 | OCT | FPhF | HEP | 709 | NON | FPhF | HEP | 714 | DEC | FPhF | OCT |
| 705 | OCT | FPhF | OCT | 710 | NON | FPhF | OCT | 715 | DEC | FPhF | NON |

Compounds of the present invention can be produced in accordance with the following reactions.

I. Compound of the formula : R-A-#-NAP-OR'(I)

[1a] In case where A is Pyr> :

$O_2N$-Pyr>-X'(2) $\xrightarrow{PtO_2, H_2}$ $H_2N$-Pyr>-X'(3) $\xrightarrow{NaNO_2, KI}$ I-Pyr>-X'(4)

(X':Cl,Br)

$\xrightarrow{R''\text{-\#-}H(5),\ [Pd]}$ R"-$CH_2CH_2$-:R-
R"-#-Pyr>-X'(6) $\xrightarrow{PtO_2, H_2}$ R-Pyr>-X' (7)

$\xrightarrow{H\text{-\#-}C(CH_3)_2OH,\ [Pd]}$ R-Pyr>-#-$C(CH_3)_2OH$(8) $\xrightarrow{NaOH}$ R-Pyr>-#-H(9)

$\xrightarrow{Br\text{-}NAP\text{-}OR'(10),\ [Pd]}$ R-Pyr>-#-NAP-OR' (1a)

A compound (3), prepared by hydrogenation of compound (2) with platinum dioxide, is diazotized with sodium nitrite, and then reacted with potassium iodide to obtain a compound(4). A compound (4) is reacted with

a compound (5) in the presence of palladium catalyst to obtain a compound (6). A compound (6) is hydrogenated in the presence of platinum dioxide to obtain a compound (7). A compound (7) is reacted with 3-methyl-1-butyn-3-ol in the presence of palladium catalyst to obtain a compound (8). A compound (8) is refluxed with sodium hydroxide in anhydrous toluene to obtain a compound (9).

A compound (10), obtained by etherifying of 6-bromo-2-naphthol with an alkylating reagent (such as alkyl halide or alkyl p-toluene sulfonate ), reacted with a compound (9) in the presence of palladium catalyst to obtain a compound (1a) of the present invention.

Compound (1a) can also be produced as follows.

$$(10) \xrightarrow{H\text{-}\#\text{-}C(CH_3)_2OH,\ [Pd]} HO(CH_3)_2\text{-}C\text{-}\#\text{-}NAP\text{-}OR'(11) \xrightarrow{NaOH}$$

$$H\text{-}\#\text{-}NAP\text{-}OR'(12) \xrightarrow{(7),\ [Pd]} (1a)$$

A compound (10) is reacted with 3-methyl-1-butyn-3-ol in the presence of palladium catalyst to obtain a compound (11). A compound (11) is refluxed with sodium hydroxide in anhydrous toluene to obtain a compound (12). Compound (1a) of the present invention can be obtained by reacting the compound (12) with the compound (7) in the presence of palladium catalyst.

Compound (1a) can also be produced as follows.

$$Br\text{-}NAP\text{-}O\overset{O}{\overset{\|}{C}}CH_3 \xrightarrow{(8),\ [Pd]} R\text{-}Pyr\text{>-}\#\text{-}NAP\text{-}O\overset{O}{\overset{\|}{C}}CH_3(13) \xrightarrow{NaOH,H^+}$$

$$R\text{-}Pyr\text{>-}\#\text{-}NAP\text{-}OH \xrightarrow{R'X(14),Base\ or R'OH(15),DEAD,triphenylphosphine} (1a)$$

(X:Cl,Br,I or Me-Ph-$SO_3$-),DEAD:diethyl azodicarboxylate

A compound (8) is reacted with a 6-bromo-2-acetoxynaphthalene in the presence of palladium catalyst to obtain a compound (13). A compound (13) is hydrolyzed and reacted with a compound (14) in the presence of base or a compound (15) in the presence of DEAD and triphenylphosphine to obtain a compound (1a) of the present invention.

[1b] In case where A is FPhF :

$$H\text{-}FPhF\text{-}H \xrightarrow{n\text{-}BuLi,R''CH_2\text{-}CHO(16)} R''CH_2\text{-}\overset{OH}{\overset{|}{C}H}\text{-}FPhF\text{-}H\ (17) \xrightarrow{H^+}$$

$$R''CH{=}CH\text{-}FPhF\text{-}H(18) \xrightarrow{H_2,Pd/C} R\text{-}FPhF\text{-}H(19) \xrightarrow{n\text{-}BuLi,I_2}$$

$$R\text{-}FPhF\text{-}I(20) \xrightarrow{H\text{-}\#\text{-}C(CH_3)_2OH,\ [Pd]} R\text{-}FPhF\text{-}\#\text{-}C(CH_3)_2OH(21) \xrightarrow{NaOH}$$

$$R\text{-}FPhF\text{-}\#\text{-}H(22) \xrightarrow{(10),\ [Pd]} R\text{-}FPhF\text{-}\#\text{-}NAP\text{-}OR'\ (1b)$$

A o-difuluorobenzen is lithiated by n-buthyl lithium followed by reacting with a compound (16) to obtain a compound (17). A compound (17) is dehydrated with acid (such as p-toluene sulufonic acid) followed by hydrogenating in the presence of palladium carbon to obtain a compound (19). A compound (19) is lithiated by n-buthyl lithium followed by reacting with iodine to obtain a compound (20). A compound (20) is reacted with 3-methyl-1-butyn-3-ol in the presence of palladium catalyst to obtain a compound (21). A compound (21) is refluxed with sodium hydroxide in anhydrous toluene to obtain a compound (22). A compound (1b) of the present invention can be obtained by reacting the compound(22) with the compound (10) in the presence of palladium catalyst.

Compound (1b) can also be produced as follows.

$$(20) \xrightarrow{(12),\ [Pd]} (1b)$$

A compound (20) is reacted with a compound (12) in the presence of palladium catalyst to obtain a compound (1b) of the present invention.

Compound (1b) can also be produced as follows.

$$\text{HO-FPhF-H} \xrightarrow{\text{NaOCl, NaI or } I_2} \text{HO-FPhF-I} \xrightarrow{\text{H-Ph-CH}_2\text{Cl, Base}}$$

$$\text{H-Ph-CH}_2\text{O-FPhF-I} \xrightarrow{\text{R''-\#-H(5), [Pd]}} \text{R''-\#-FPhF-OCH}_2\text{-Ph-H (23)}$$

$$\xrightarrow{H_2\text{, Pd/C}} \text{R-FPhF-OH(24)} \xrightarrow{\text{trifluoromethanesulfonic anhydride(TFMSA)}}$$

$$\text{R-FPhF-OTf(25)} \xrightarrow{(12),\ [Pd]} (1b)$$

$$\text{TfO-:CF}_3\text{SO}_3\text{-}$$

A 2,3-difluoro-4-iodophenol is obtained by iodination of 2,3-difluorophenol using a sodium hypochlorite and sodium iodode or iodine. A 2,3-difluoro-4-benzyloxyiodobenzene is obtained by reacting a 2,3-difluoro-4-iodophenol and benzyl chloride in the presence of base (such as sodium hydroxide). A compound (23) is obtained by reacting a 2,3-difluoro-4-benzyloxyiodobenzene and a compound (5) in the presence of palladium catalyst. Then the compound (23) is hydrogenated and hydrogenolyzed using palladium carbon into a compound (24). A compound (25) prepared by reacting the compound (24) with TFMSA is reacted with a compound (12) in the presence of palladium catalyst to obtain a compound (1b) of this invention.

[1c] In case where A is FPh :

$$\text{I-FPh-Br} \xrightarrow{(5),\ [Pd]} \text{R''-\#-FPh-Br(26)} \xrightarrow{PtO_2\text{, }H_2} \text{R-FPh-Br(27)}$$

$$\xrightarrow{\text{H-\#-C(CH}_3)_2\text{OH, [Pd]}} \text{R-FPh-\#-C(CH}_3)_2\text{OH(28)} \xrightarrow{\text{NaOH}} \text{R-FPh-\#-H(29)}$$

$$\xrightarrow{(10),\ [Pd]} \text{R-FPh-\#-NAP-OR' (1c)}$$

A compound (26) is obtained by reacting a 1-bromo-3-fluoro-4-iodobenzene and a compound (5) in the presence of palladium catalyst. Then the compound (26) is hydrogenated using platinum dioxide into a compound (27). A compound (28) prepared by reacting a compound (27) and 3-methyl-1-butyn-3-ol in the presence of palladium catalyst is refluxed with sodium hydroxide in anhydrous toluene to obtain a compound (29).

A compound (1c) of the present invention can be obtained by reacting the compound(29) with the compound (10) in the presence of palladium catalyst.

Compound (1c) can also be produced as follows.

(27) —(12), [Pd]→ (1c)

A compound (27) is reacted with a compound (12) in the presence of palladium catalyst to obtain a compound (1c) of this invention.

II- Compound of the formula : R-O-A-#-NAP-R′(II)

[2a] In case where A is FPhF :

Br-NAP-$OCH_2$-Ph-H —R'''-#-H(5'), [Pd] R'''-$CH_2CH_2$-:R'→ R'''-#-NAP-$OCH_2$-Ph-H(30)

—$H_2$,Pd/C→ R'-NAP-OH(31) —TFMSA→ R'-NAP-OTf(32) —H-#-$C(CH_3)_2OH$, [Pd]→

R'-NAP-#-$C(CH_3)_2OH$(33) —NaOH→ R'-NAP-#-H (34) —I-FPhF-OR(35), [Pd]→

RO-FPhF-#-NAP-R' (2a)

A compound (30) is obtained by reacting a compound (5′) and 6-bromo-2-benzyloxynaphthalene in the presence of palladium catalyst. Then the compound (30) is hydrogenated and hydrogenolyzed using palladium carbon into a compound (31). A compound (32) is obtained by reacting a compound (31) and TFMSA. A compound (33) prepared by reacting a compound (32) and 3-methyl-1-butyn-3-ol in the presence of palladium catalyst is refluxed with sodium hydroxide in anhydrous toluene to obtain a compound (34). A compound (2a) of the present invention can be obtained by reacting the compound(34) with the compound (35), obtained by etherifying of 2,3-difluoro-4-iodophenol with an alkylating reagent (such as alkyl halide or alkyl p-toluene sulufonate) in the presence of palladium catalyst.

Compound (2a) can also be produced as follows.

(35) —H-#-$C(CH_3)_2OH$, [Pd]→ RO-FPhF-#-$C(CH_3)_2OH$(36) —NaOH→

RO-FPhF-#-H(37) —(32), [Pd]→ (2a)

A compound (36) prepared by reacting a compound (35) and 3-methyl-1-butyn-3-ol in the presence of palladium catalyst is refluxed with sodium hydroxide in anhydrous toluene to obtain a compound (37). A compound (2a) of the present invention can be obtained by reacting the compound(37) with the compound (32) in the presence of palladium catalyst.

Compound (2a) can also be produced as follows.

H-FPhF-H —n-BuLi,$I_2$→ H-FPhF-I —(34), [Pd]→ H-FPhF-#-NAP-R'(38)

—n-BuLi,$B(OMe)_3$,$H^+$→ $(HO)_2B$-FPhF-#-NAP-R'(39) —$H_2O_2$→

HO-FPhF-#-NAP-R'(40) —RX(14'),Base orROH(15'),DEAD,triphenylphosphine→ (2a)

A o-difuluorobenzen is lithiated by n-buthyl lithium followed by reacting with iodine to obtain a 2,3-difluoroiodobenzene.

A compound (38) is obtained by reacting a 2,3-difluoro iodobenzene and a compound (34) in the presence of palladium catalyst. A compound (38) is lithiated by n-buthyl lithium and then reacting with trimethyl borate followed by hydrolysis with an acid to obtain a compound (39). A compound (39) is reacted with hydrogen peroxide to obtain a compound (40). A compound (40) is reacted with a compound (14′) in the presence of base or a compound (15′) in the presence of DEAD and triphenylphosphine to obtain a compound (2a) of the present invention.

[2b] In case where A is FPh or PhF:

$$\text{HO-Ph(F)-Br(41)} \xrightarrow{(14')\text{ or }(15')} \text{RO-Ph(F)-Br(42)} \xrightarrow{\text{H-\#-C(}CH_3)_2\text{OH, [Pd]}}$$

$$\text{RO-Ph(F)-\#-C(}CH_3)_2\text{OH(43)} \xrightarrow{\text{NaOH}} \text{RO-Ph(F)-\#-H(44)} \xrightarrow{\text{(32), [Pd]}}$$

$$\text{RO-Ph(F)-\#-NAP-R'}\quad\text{(2b)}$$

A compound (41) is reacted with a compound (14′) in the presence of base or a compound (15′) in the presence of DEAD and triphenylphosphine to obtain a compound (42). A compound (43) prepared by reacting a compound (42) and 3-methyl-1-butyn-3-ol in the presence of palladium catalyst is refluxed with sodium hydroxide in anhydrous toluene to obtain a compound (44). A compound (2b) of the present invention can be obtained by reacting the compound(44) with the compound (32) in the presence of palladium catalyst.

Compound (2b) can also be produced as follows.

$$\text{(42)} \xrightarrow{\text{(34), [Pd]}} \text{(2b)}$$

A compound (2b) of the present invention can be obtained by reacting the compound(42) with the compound (34) in the presence of palladium catalyst.

Compound (2b) can aiso be produced as follows.

$$CH_3\overset{O}{C}\text{O-Ph(F)-Br(45)} \xrightarrow{\text{(34), [Pd]}} CH_3\overset{O}{C}\text{O-Ph(F)-\#-NAP-R'(46)} \xrightarrow{\text{NaOH,}H^+}$$

$$\text{HO-Ph(F)-\#-NAP-R'(47)} \xrightarrow{(14')\text{ or }(15')} \text{(2b)}$$

A compound (45) is reacted with a compound (34) in the presence of palladium catalyst to obtain a compound (46). A compound (46) is hydrolyzed and reacted with a compound (14′) in the presence of base or a compound (15′) in the presence of DEAD and triphenylphosphine to obtain a compound (2b) of the present invention.

III. Compound of the formula : R-O-A-#-NAP-O-R′(III)

[3a] In case where A is FPhF :

$$\text{(10)} \xrightarrow{\text{(37), [Pd]}} \text{RO-FPhF-\#-NAP-OR'}\quad\text{(3a)}$$

A compound (3a) of the present invention can be obtained by reacting the compound(10) with the compound (37) in the presence of palladium catalyst.

Compound (3a) can also be produced as follows.

$$(12) \xrightarrow{(35),\ [Pd]} (3a)$$

A compound (3a) of the present invention can be obtained by reacting the compound(12) with the compound (35) in the presence of palladium catalyst.

Compound (3a) can also be produced as follows.

$$Br\text{-}NAP\text{-}O\overset{O}{\overset{\|}{C}}CH_3 \xrightarrow{(37),\ [Pd]} RO\text{-}FPhF\text{-}\#\text{-}NAP\text{-}O\overset{O}{\overset{\|}{C}}CH_3\ (48) \xrightarrow{NaOH,H^+}$$

$$RO\text{-}FPhF\text{-}\#\text{-}NAP\text{-}OH\ (49) \xrightarrow{(14)\ or\ (15)} (3a)$$

A compound (37) is reacted with a 6-bromo-2-acetoxynaphthalene in the presence of palladium catalyst to obtain a compound (48).

A compound (48) is hydrolyzed and reacted with a compound (14) in the presence of base or a compound (15) in the presence of DEAD and triphenylphosphine to obtain a compound (3a) of the present invention.

Compound (3a) can also be produced as follows.

$$H\text{-}FPhF\text{-}I \xrightarrow{(12),\ [Pd]} H\text{-}FPhF\text{-}\#\text{-}NAP\text{-}OR'(50) \xrightarrow{n\text{-}BuLi,B(OMe)_3,H^+}$$

$$(HO)_2B\text{-}FPhF\text{-}\#\text{-}NAP\text{-}OR'(51) \xrightarrow{H_2O_2} HO\text{-}FPhF\text{-}\#\text{-}NAP\text{-}OR'(52)$$

$$\xrightarrow{(14')\ or\ (15')} (3a)$$

A compound (50) is obtained by reacting a 2,3-difluoro iodobenzene and a compound (12) in the presence of palladium catalyst. A compound (50) is lithiated by n-buthyl lithium and then reacting with trimethyl borate followed by hydrolysis with an acid to obtain a compound (51). A compound (51) is reacted with hydrogen peroxide to obtain a compound (52). A compound (52) is reacted with a compound (14′) in the presence of base or a compound (15′) in the presence of DEAD and triphenylphosphine to obtain a compound (3a) of the present invention.

[3b] In case where A is FPh :

$$HO\text{-}FPh\text{-}Br\ (41') \xrightarrow{(14')\ or\ (15')} RO\text{-}FPh\text{-}Br(42') \xrightarrow{H\text{-}\#\text{-}C(CH_3)_2OH,\ [Pd]}$$

$$RO\text{-}FPh\text{-}\#\text{-}C(CH_3)_2OH(43') \xrightarrow{NaOH} RO\text{-}FPh\text{-}\#\text{-}H(44') \xrightarrow{(10),\ [Pd]}$$

$$RO\text{-}FPh\text{-}\#\text{-}NAP\text{-}OR'\quad (3b)$$

A compound (41′) is reacted with a compound (14′) in the presence of base or a compound (15′) in the presence of DEAD and triphenylphosphine to obtain a compound (42′). A compound (43′) prepared by reacting a compound (42′) and 3-methyl-1-butyn-3-ol in the presence of palladium catalyst is refluxed with sodium hydroxide in anhydrous toluene to obtain a compound (44′).

A compound (3b) of the present invention can be obtained by reacting the compound(44′) with the compound (10) in the presence of palladium catalyst.

[3c] In case where A is <Pyr :

RONa(53) —(Br-<Pyr-Br)→ RO-<Pyr-Br(54) —(H-#-$C(CH_3)_2OH$, [Pd])→

RO-<Pyr-#-$C(CH_3)_2OH$(55) —(NaOH)→ RO-<Pyr-#-H(56) —((10), [Pd])→

RO-<Pyr-#-NAP-OR' (3c)

A compound (54) is obtained by reacting a compound (53) and 2,5-dibromopyridine. A compound (55) prepared by reacting a compound (54) and 3-methyl-1-butyn-3-ol in the presence of palladium catalyst is refluxed with sodium hydroxide in anhydrous toluene to obtain a compound (56). A compound (3c) of the present invention can be obtained by reacting the compound(56) with the compound (10) in the presence of palladium catalyst.

Compound (3c) can also be produced as follows.

(54) —((12), [Pd])→ (3c)

A compound (3c) of the present invention can be obtained by reacting the compound (54) with the compound (12) in the presence of palladium catalyst.

IV. Compound of the formula : R-O-A-#-NAP-COO-R'(IV)

[4a] In case where A is FPhF, EPh or <Pyr :

H-Ph-$CH_2$O-NAP-$\overset{O}{C}$OH —((15))→ H-Ph-$CH_2$O-NAP-$\overset{O}{C}$OR'(57) —($H_2$,Pd/C)→

HO-NAP-$\overset{O}{C}$OR'(58) —(TFMSA)→ TfO-NAP-$\overset{O}{C}$OR'(59) —((37),(44')or(56) [Pd])→

RO-A-#-NAP-$\overset{O}{C}$OR' (4a)

A compound (57) prepared by reacting a 6-benzyloxy-2-naphthoic acid and a compound (15) in the presence of DEAD and triphenylphosphine or using thionyl chloride is hydrogenolyzed using palladium carbon into a compound (58). A compound (59) prepared by reacting a compound (58) and TFMSA is reacted with a compound (37) [(44') or(56)] in the presence of palladium catalyst to obtain a compound (4a) of the present invention.

Compound (4a) can also be produced as follows.

TfO-NAP-COOEt(60) —((37),(44')or(56) [Pd])→ RO-A-#-NAP-$\overset{O}{C}$OEt (61)

—(NaOH,$H^+$)→ RO-A-#-NAP-$\overset{O}{C}$OH(62) —((15))→ (4a)

A compound (61) prepared by reacting a compound (60) with a compound (37) [(44') or (56)] in the presence of palladium catalyst is hydrolyzed and then reacted with a compound (15) in the presence of DEAD and triphenylphosphine or using thionyl chloride to obtain a compound (4a) of the present invention.

V. Compound of the formula : R-A-#-NAP-COO-R'(V)

[5a] In case where A is FPhF, EPh or Pyr> :

$$(59)\ \xrightarrow{(9),(22)\,or\,(29)\ [Pd]}\ R\text{-}A\text{-}\#\text{-}NAP\text{-}\overset{O}{\overset{\|}{C}}OR'\ (5a)$$

A compound (59) is reacted with a compound (9) [(22) or(29)] in the presence of palladium catalyst to obtain a compound (5a) of the present invention.

Compound (5a) can also be produced as follows.

$$(60)\ \xrightarrow{(9),(22)\,or\,(29)\ [Pd]}\ R\text{-}A\text{-}\#\text{-}NAP\text{-}\overset{O}{\overset{\|}{C}}OEt\ (63)\ \xrightarrow{NaOH,\,H^+}$$

$$R\text{-}A\text{-}\#\text{-}NAP\text{-}\overset{O}{\overset{\|}{C}}OH\ (64)\ \xrightarrow{(15)}\ (5a)$$

A compound (63) prepared by reacting a compound (60) with a compound (9) [(22) or (29)] in the presence of palladium catalyst is hydrolyzed and then reacted with a compound (15) in the presence of DEAD and triphenylphosphine to obtain a compound (5a) of the present invention.

VI. Compound of the formula : R-OCO-A-#-NAP-R'(VI)

[6a] In case where A is Pyr> :

$$HO\overset{O}{\overset{\|}{C}}\text{-}Pyr\text{>-}Cl\ \xrightarrow{(15')}\ RO\overset{O}{\overset{\|}{C}}\text{-}Pyr\text{>-}Cl\,(65)\ \xrightarrow{(34)\ [Pd]}$$

$$RO\overset{O}{\overset{\|}{C}}\text{-}Pyr\text{>-}\#\text{-}NAP\text{-}R'\ (6a)$$

A compound (65) prepared by reacting a 2-chloronicotinic acid and a compound in the presence of DEAD and triphenylphosphine is reacted with a compound (34) in the presence of palladium catalyst to obtain a compound (6a) of the present invention.

[6b] In case where A is FPhF :

$$(38)\ \xrightarrow{n\text{-}BuLi,\,CO_2\ ,\,H^+}\ HO\overset{O}{\overset{\|}{C}}\text{-}FPhF\text{-}\#\text{-}NAP\text{-}R'(66)\ \xrightarrow{(15')}$$

$$RO\overset{O}{\overset{\|}{C}}\text{-}FPhF\text{-}\#\text{-}NAP\text{-}R'\ (6b)$$

A compound (38) is lithiated by n-buthyl lithium and then reacting with carbon dioxide followed by hydrolysis with an acid to obtain a compound (66). A compound (66) is reacted with a compound (15') in the presence of DEAD and triphenylphosphine or using a thionyl chloride to obtain a compound (6b) of the present invention.

[6c] In case where A is FPh :

$$\text{Me-FPh-I} \xrightarrow{KMnO_4} \text{HO}\overset{O}{\overset{\|}{C}}\text{-FPh-I} \xrightarrow{(15')} \text{RO}\overset{O}{\overset{\|}{C}}\text{-FPh-I (67)} \xrightarrow{(34)\ [Pd]}$$

$$\text{RO}\overset{O}{\overset{\|}{C}}\text{-FPh-\#-NAP-R'} \quad (6c)$$

A 2-fluoro-4-iodotoluene is oxidized by potassium permanganate to obtain a 2-fluoro-4-iodobenzoic acid. A 2-fluoro-4-iodobenzoic acid is reacted with a compound (15′) in the presence of DEAD and triphenylphosphine or using a thionyl chloride to obtain a compound (67). A compound (67) is reacted with a compound (34) in the presence of palladium catalyst to obtain a compound (6c) of the present invention.

VII. Compound of the formula : R-OCO-A-#-NAP-OR′(VII)

[7a] In case where A is Pyr> :

$$\text{(65)} \xrightarrow{(12)\ [Pd]} \text{RO}\overset{O}{\overset{\|}{C}}\text{-Pyr>-\#-NAP-OR'} \quad (7a)$$

A compound (65) is reacted with a compound (12) in the presence of palladium catalyst to obtain a compound (7a) of the present invention.

[7b] In case where A is FPhF :

$$\text{(50)} \xrightarrow{n\text{-}BuLi,\ CO_2\ ,\ H^+} \text{HO}\overset{O}{\overset{\|}{C}}\text{-FPhF-\#-NAP-OR'(68)} \xrightarrow{(15')}$$

$$\text{RO}\overset{O}{\overset{\|}{C}}\text{-FPhF-\#-NAP-OR'} \quad (7b)$$

A compound (50) is lithiated by n-buthyl lithium and then reacting with carbon dioxide followed by hydrolysis with an acid to obtain a compound (68). A compound (68) is reacted with a compound (15′) in the presence of DEAD and triphenylphosphine to obtain a compound (7b) of the present invention.

[7c] In case where A is FPh :

$$\text{RO}\overset{O}{\overset{\|}{C}}\text{-FPh-I (67)} \xrightarrow{(12)\ [Pd]} \text{RO}\overset{O}{\overset{\|}{C}}\text{-FPh-\#-NAP-OR'} \quad (7c)$$

A compound (67) is reacted with a compound (12) in the presence of palladium catalyst to obtain a compound (7c) of the present invention.

VIII. Compound of the formula : R-A-#-NAP-R′(VIII)

[8a] In case where A is FPhF :

$$\text{(34)} \xrightarrow{(20)\text{or}(25)\ [Pd]} \text{R-FPhF-\#-NAP-R'} \quad (8a)$$

A compound (34) is reacted with a compound (20) or compound (25) in the presence of palladium catalyst

to obtain a compound of the present invention.

Compound (8a) can also be produced as follows.

$$(32) \xrightarrow{(22)\ [Pd]} (8a)$$

A compound (32) is reacted with a compound (20) in the presence of palladium catalyst to obtain a compound of the present invention.

In general, liquid crystals are used in the forms of liquid crystal compositions containing two or more components; and compounds of the present invention can be used as a component of liquid crystal compositions. Liquid crystal composition according to the present invention comprises a mixture of a pluralty of compounds, comprising at least one compound of this invention. Liquid crystal compositions of the invention include [1] ones showing Sc phase, and [2] ones showing Sc∗ phase.

Liquid crystal compositions [1] of the present invention comprise at least one liquid crystal compound of this invention as the essential component, and can optionally contain one or more other liquid crystal compounds, which may be selected from the group consisting of other liquid crystal compounds showing Sc phase, liquid crystal compounds showing smectic phase other than Sc or Sc∗ phase, and liquid crystal compounds showing nematic phase. These liquid crystal compounds include, for example, those written in EP 220,296. Illustrative of suitable liquid crystal compounds showing Sc phase are 2-4′-alkoxyphenyl-5-alkylpyrimidine, 2-4′-alkylphenyl-5-alkoxypyrimidine, 2-4′-alkoxyphenyl-5-alkoxypyrimidine, 2-4′-alkylphenyl-5-alkylpyrimidine, 2-p-alkoxycarbonylphenyl-5-alkylprimidine, 2-4′-alkoxy-3′-fluorophenyl-5-alkylpyrimidine, 2-4′-alkoxy-2′,3′-difluorophenyl-5-alkylpyrimidine, 2-4′-alkoxyphenyl-5-alkylpyridine, 2-4′-alkoxy-3′-fluorophenyl-5-alkylpyridine, 2-4′-alkoxy-2′,3′-difluorophenyl-5-alkylpyridine, 2-4′-alkylbiphenyl-5-alkylpyrimidine, 2-4′-alkoxybiphenyl-5-alkylpyrimidine, 2-4′-alkoxybiphenyl-5-alkoxypyrimidine, 2-4′-alkylbiphenyl-5-alkylpyrimidine, 2-4′-alkylphenyl-5-4′-alkoxyphenylpyrimidine, 2-4′-alkoxyphenyl-5-4′-alkoxyphenylpyrimidine, 2-4′-alkoxyphenyl-5-4′-alkylphenylpyrimidine, 2-4′-alkylphenyl-5-4′-alkoxyphenylpyrimidine, 1-5′-alkyl-2′-pyridyl-2-4′-alkoxyphenylacetylene, 1-2′-alkyl-5′-pyridyl-2-4′-alkoxyphenylacetylene, 1-2′-alkyl-5′-pyridyl-2-4′-alkoxy-3′-fluorophenylacetylene, 1-2′-alkyl-5′-pyridyl-2-4′-alkoxy-2′,3′-difluorophenylacetylene, 1-3′-alkyl-5′-pyridazyl-2-4′-alkoxy-3′-fluorophenylacetylene, and 1-3′-alkoxy-5′-pyridazyl-2-4′-alkoxy-3′-fluorophenylacetylene.

Liquid crystal compositions [2] of the present invention comprise at least one liquid crystal compound of this invention as the essential component and optionally one or more other liquid crystal compounds, and contain at least one optically active liquid crystal compound which may be the compound of this invention or/and the other liquid crystal compound. Liquid crystal compositions [2] include, for example, ones comprising said liquid crystal composition [1] and at least one optically active compound, such as liquid crystal compounds showng Sc∗ phase and chiral compounds. Illustrative examples of suitable other liquid crystal compounds include ones showing Sc∗ phase, for example, oa p′-(2-methylbutyloxycarbonyl)phenyl 4-alkoxy-4′-bi-phenylcarboxylate, oa 2-methylbutyl 4-alkoxy-4′-biphenyl-carboxylate; oa 2-(2-methylbutyloxycarbonyl)naphthalene-6-4′-n-alkoxyphenylcarboxylate, oa 2-(1-methylheptyloxy-carbonyl)naphthalene-6-4′-n-alkoxyphenylcarboxylate, oa 2-(2-methylbutyloxy)naphthalene-6-4′-n-alkoxyphenylcarboxyl-ate and oa 2-(1-methylheptyloxy)naphthalene-6-4′-n-alkoxy-phenylcarboxylate; and other optically active compounds, for instance, those written in JPN Patent Lay-open Nos. 99032/1988, 190843/1988, 138274/1990 and 27374/1991, and EP Patents 374,789, 365,820 and 395,078 (corresponding to JPN Patent Lay-open Nos.256673/ 1990, 262579/1990 and 286673/1990, respectively). Illustrative of these compounds are oa 2-(1-cyanoethyl)naphthalene-6-4′-n-alkyl-phenylcarboxylate, 2-(1-cyanoethyl)naphthalene-6-4′-n-alkoxyphenylcarboxylate, oa 4-(1-trifluoromethylheptyloxy-methyl)-4′-n-alkoxy-biphenyl, oa 4-(1-trifluoromethylheptyl-oxymethyl)-4′-n-alkylbiphenyl, oa trans 4-(4′-n-alkoxy-phenyl)-1-(1′-trifluoromethylheptyloxycarbonyl)cyclo-hexane, and the like.

Liquid crystal compositions may contain other components, for example, chiral compounds showing no liquid crystaline properties, pleochroic dyes (such as anthraquinone dyes, azo dyes and the like), and so on.

Content of said liquid crystal compound of this invention in liquid crystal compositions of the invention, which is not particularly restricted and can be varied widely, is usually 5-100 % by weight (5-99% in case of compositions [2]). Content of pleochroic dye is usually 0-5 % by weight.

By applied voltage, ferroelectric liquid crystal compositions of this invention cause photo-switching phenomena, which are applied for producing display elements of quick response [See for example, JPN Patent Lay-open Nos.107216/1981 and 118744/1984, and N.A.Clark and S.T.Lagerwall:Applied Physics Letters,36,899(1980)].

Liquid crystal compositions, showing ferroelectricity, according to the invention, bring about optical switching phenomena by applied voltage, and can provide display devices, such as those described in JPN Lay-open

Patents No.107216/1981 and No.118744/1984, and N.A.Clark and S.T.Lagerwall, "Applied Physics Letters" 36,899(1980).

The liquid crystal compositions of this invention can be used as photo-switching elements (display devices), for instance, by sealing in vacuum into liquid crystal cells having cell distance of 0.5-10 (preferably 0.5-3) micron m, followed by providing polarizers on both sides.

The above liquid crystal cells can be produced by combining, through a spacer, two surface-aligned glass substrates provided with transparent electrodes. Examples of suitable spacers are alumina beads, glass fiber and polyimide film. Alignment can be done by conventional alignment treatment, such as application of polyimide membrane, rubbing treatment, oblique vapor-deposition of SiO.

Having generally described the invention, a more complete understanding can be obtained by reference to certain specific examples, which are included for purposes of illustration only and not intended to be limiting unless.otherwise specified. In the following Examples, % represents % by weight.

Example 1 [Compound No.10]

(1) Into 250 ml of dimethylsulfoxide (hereinafter referred to as DMSO) containing 25.0 g of 6-bromo-2-naphthol dissolved therein, were added 20 ml of an aqueous solution of 4.9 g of sodium hydroxide, and stirred into a homogeneous solution. Then, 22.7 g of n-octyl bromide were added and stirred for 3 days at room temperature. Thereafter, the reaction product was thrown into iced water, and the resulting precipitates were separated by suction filtration, and recrystalize with methanol to obtain 33.4 g of 2-n-octyloxy-6-bromonaphthalene.

(2) In 100 ml of triethylamine, 10.0 g of 2-n-octyloxy-6-bromonaphthalene and 3.0 g of 3-methyl-1-butyne-3-ol were heated to reflux for 6 hours within an atmosphere of nitrogen in the presence of 25 mg of cuprous iodide (I), 100 mg of dichlorobistriphenylphosphine palladium(II) and 200 mg of triphenylphosphine. After reaction, the temperature was reduced to the room temperature and the triethyl amine was removed. After the product was extracted with toluene, the toluene layer was washed with 1N-hydrochloric acid and then water, followed by removing the toluene. The product was dissolved in methanol and treated with activated carbon, followed by removing the methanol. To the resultant product, were added 300 ml of drytoluene and 2.4 g of sodium hydroxide powder, followed by heating to reflux for an hour. After cooling, the toluene layer was washed with water, followed by removing the toluene. The resulting solid was recrystalized with methanol to obtain 5.4 g of 2-n-octyloxy-6-ethynyl-naphthalene.

(3) In 200 ml of ethanol, within hydrogen atmosphere, 20.0 g of 2-bromo-5-nitropyridine were reduced with use of 1.0 g of platinum dioxide. After comfirming that no absorption of hydrogen was observed, the catalyst was filtered off. After removing the ethanol, the product was recrystalized with toluene to obtain 17.4 g of 5-amino-2-bromopyridine.

(4) To 900 ml of water, were added 16.0 g of 5-amino-2-bromopyridine and 95 g of 35% hydrochloric acid, and cooled to a temperature below 10°C. Thereto, were added dropwise 20 ml of an aqueous solution containing 6.4 g of sodium nitrite, follwed by continuing stirring for an hour in that condition. To this aqueous solution, was added an aqueous solution containing 30 g of potassium iodide, and stirred until no nitrogen was generated. After confirming that no generation of nitrogen was observed, the product was neutralized with sodium hydroxide, followed by adding sodium thiosulfate and stirring for an hour. The resulting precipitates were dried and then recrystalized with hexane to obtain 20.0 g of 2-bromo-5-iodopyridine.

(5) In 150 ml of triethylamine, within nitrogen atmosphere, 20.0 g of 2-bromo-5-iodopyridine and 8.8 g of 1-nonyne were reacted for 5 hours at the room temperature in the presence of 240 mg of dichlorobistriphenylphosphine palladium(II) and 60 mg of cuprous iodide (I) as catalysts. After reaction, the triethyl amine was removed, and the product was extracted with hexane. The hexane layer was washed with IN-hydrochloric acid and then water, followed by removing the hexane. The product was dissolved in ethanol and hydrogenated within hydrogen atmosphere using platinum dioxide. After comfirming that no absorption of hydrogen was observed, the catalyst was filtered off, follwed by removing the ethanol. The product was dissolved in hexane to remove impuritiese, and then the hexane was removed to obtain 8.7 g of liquid 2-bromo-5-n-nonylpyridine.

(6) In 40 ml of triethylamine, within nitrogen atmosphere, 1.5 g of 2-n-octyloxy-6-ethynyl-naphthalene and 1.7 g of 2-bromo-5-n-nonylpyridine were reacted for 6 hours at reflux temperature in the presence of 20 mg of dichlorobistriphenylphosphine palladium(II), 5 mg of cuprous iodide and 40 mg of triphenylphosphine. After reaction, the triethylamine was removed, and the product was extracted with toluene. The toluene layer was washed with 1N-hydrochloric acid and then with water, and treated with silica gel column, followed by removing the toluene. The resultant solid was recrystalized three times with ethanol to obtain 1.3 g of Compound No.10 of the present invention, the structure of which was confirmed by IR and NMR spectra and elemental analysis.

IR ($cm^{-1}$) 2926 2856 2214 1624 1603 1464 1388 1261 1209 1174 895 861 812 476

NMR (ppm) 0.84-0.94(m,6H) 1.20-1.42(m,20H) 1.42-1.55 (m,2H) 1.55-1.68(m,2H) 1.78-1.89(m,2H) 2.61 (t,2H) 4.07(t,2H) 7.09(d,1H) 7.16(dd,1H) 7.45-7.50(m,2H) 7.57(dd,1H) 7.69(t,2H) 8.02(s,1H) 8.45(s,1H)

| | | | | |
|---|---|---|---|---|
| Elemental analysis,% | Observed | C: 80.93 | H: 9.42 | N: 5.97 |
| | Theoretical | C: 80.87 | H: 9.57 | N: 6.09 |

Example 2 [Compound No.9]

(1) Example 1 (1),(2) was repeated except that n-octyl bromide was substituted with n-heptyl bromide to obtain 2-n-heptyloxy-6-ethynylnaphthalene.
(2) Example 1 (6) was repeated except that 2-n-octyloxy-6-ethynylnaphthalene was substituted with 2-n-heptyloxy-6-ethynylnaphthalene to obtain Compound No.9.

Example 3 [Compound No.8]

(1) Example 1 (1),(2) was repeated except that n-octyl bromide was substituted with n-hexyl bromide to obtain 2-n-hexyloxy-6-ethynylnaphthalene.
(2) Example 1 (6) was repeated except that 2-n-octyloxy-6-ethynylnaphthalene was substituted with 2-n-hexyloxy-6-ethynylnaphthalene to obtain Compound No.8.

Example 4 [Compound No.4]

(1) Example 1 (5) was repeated except that 1-nonyne was substituted with 1-octyne to obtain 2-bromo-5-octylpyridine.
(2) Example 1 (6) was repeated except that 2-n-octyloxy-6-ethynyl-naphthalene and 2-bromo-5-n-nonylpyridine were substituted with 2-n-heptyloxy-6-ethynyl-naphthalene and 2-bromo-5-n-octylpyridine, respectively, to obtain Compound No.4.

Example 5 [Compound No.5]

Example 1 (6) was repeated except that 2-bromo-5-n-nonylpyridine was substituted with 2-bromo-5-n-octylpyridine to obtain Compound No.5.

Example 6 [Compound No.12]

(1) Example 1 (5) was repeated except that 1-nonyne was substituted with 1-decyne to obtain 2-bromo-5-decylpyridine.
(2) Example 1 (6) was repeated except that 2-n-octyloxy-6-ethynyl-naphthalene and 2-bromo-5-n-nonylpyridine were substituted with 2-n-hexyloxy-6-ethynyl-naphthalene and 2-bromo-5-n-decylpyridine, respectively, to obtain Compound No.12.

Example 7 [Compound No.13]

Example 1 (6) was repeated except that 2-n-octyloxy-6-ethynyl-naphthalene and 2-bromo-5-n-nonylpyridine were substituted with 2-n-heptyloxy-6-ethynylnaphthalene and 2-bromo-5-n-decylpyridine, respectively, to obtain Compound No.13.

Example 8 [Compound No.26]

(1) Example 1 (1),(2) was repeated except that n-octyl bromide was substituted with n-nonyl bromide to obtain 2-n-nonyloxy-6-ethynylnaphthalene.
(2) To a solution of 10.0 g of 2,3-difluorophenol, 11.5 g of sodium iodide and 3.1 g of sodium hydroxide dissolved in 200 ml of methanol, were added dropwise at a temperature of 0°C 135 ml of 4% aqueous solution of sodium hypochlorite, followed by continuing stirring for 2 hours in that condition. After reaction, 80 ml of 10% aqueous solution of sodium thiosulfate were added, followed by adding 4N-hydrochloric acid into weakly acidic pH. The separated oil was extracted with chloroform, and the organic layer was washed with water, followed by removing the chloroform. The resulting oil was recrystalized with hexane to obtain 8.7 g of white solid 2,3-difluoro-4-iodophenol.

(3) To 70 ml of DMSO containing 8.7 g of 2,3-difluoro-4-iodophenol dissolved therein, was added a solution of 1.4 g of sodium hydroxide dissolved in 5 ml of water, and stirred into a homogeneous solution. Then, 4.5 g of benzyl chloride were added and stirred for 3 days at room temperature. After reaction, water was added to the product, which was then extracted with hexane. The hexane layer was washed with water, and the hexane was removed. The resultant-solid was recrystalized with methanol to obtain 10.1 g of 2,3-difluoro-4-benzyloxyiodobenzene.

(4) In 100 ml of triethylamine, within nitrogen atmosphere, 12.0 g of 2,3-difluoro-4-benzyloxyiodobenzene and 4.3 g of 1-nonyne were reacted for 16 hours at the room temperature in the presence of 30 mg of cuprous iodide(I) and 120 mg of dichlorobistriphenylphosphine palladium(II) as catalysts. After reaction, the triethyl amine was removed, and the product was extracted with toluene. The toluene layer was washed with 1N-hydrochloric acid and then water, and treated with silica gel column. After removing the toluene, 100 ml of ethanol and 200 mg of 5% palladium carbon were added to the resultant oil, and hydrogenation and hydrogenolysis were carried out at reflux temperature. After comfirming that no absorption of hydrogen was observed, the catalyst was filtered off, follwed by removing the ethanol to obtain 6.8 g of 2,3-difluoro-4-n-nonylphenol.

(5) Into 100 ml of pyridine, were dissolved 6.8 g of 2,3-difluoro-4-n-nonylphenol, and 10.0 g of trifluoromethanesulfonic anhydride were added thereto at a temperature below 10°C, followed by continuing stirring for 1 day at the room temperature. Thereafter, the reaction product was thrown into iced water and extracted with toluene. The toluene layer was washed with 1N-hydrochloric acid and then with water, followed by romoving the toluene to obtain 8.8 g of oily 2,3-difluoro-4-nonylphenyl trifluoromethanesulfonate.

(6) In 30 ml of triethylamine, within nitrogen atmosphere, 1.0 g of 2-n-nonyloxy-6-ethynylnaphthalene and 1.3 g of 2,3-difluoro-4-nonylphenyl trifluoromethanesulfonate were heated for 24 hours to reflux temperature in the presence of 3 mg of cuprous iodide and 12 mg of dichlorobistriphenylphosphine palladium(II) and 24 mg of triphenylphosphine. After reaction, the temperature was reduced to the room temperature, and the triethyl amine was removed. The product was extracted with toluene, and the toluene layer was washed with 1N-hydrochloric acid and then with water, and treated with silica gel column. After removing the toluene, the product was recrystalized three times with ethanol to obtain 0.8 g of white crystalline Compound No.26 of the present invention.

IR ($cm^{-1}$) 2920 2852 2212 1620 1603 1468 1394 1259 1218 1017 861 472

NMR (ppm) 0.88-0.94(m,6H) 1.18-1.45(m,22H) 1.45-1.55 (m,2H) 1.55-1.68(m,2H) 1.78-1.90(m,2H) 2.67 (t,2H) 4.08(t,2H) 6.88-6.94(m,1H) 7.09-7.25 (m,3H) 7.55(dd,1H) 7.70(t,2H) 7.98(d,1H)

$^{19}F$-NMR(ppm) -59.9(dd,1F) -67.4(dd,1F)

| | | | | |
|---|---|---|---|---|
| Elemental | Observed | C: 81.05 | H: 8.81 | N: 7.31 |
| analysis,% | Theoretical | C: 81.20 | H: 8.65 | N: 7.14 |

Example 9 [Compound No.144]

(1) Into 500 ml of DMSO containing 50 g of 6-bromo-2-naphthol dissolved therein, were added 20 ml of an aqueous solution of 9.8 g of sodium hydroxide, and stirred into a homogeneous solution. Then, 32 g of benzyl chloride were added devided into three parts, and stirred for 3 days at room temperature. Thereafter, the reaction product was thrown into iced water, and the resulting precipitates were filtered, dried and then recrystalized with ethanol to obtain 65.0 g of 2-benzyloxy-6-bromonaphthalene.

(2) In 100 ml of triethylamine, 15.0 g of 2-benzyloxy-6-bromonaphthalene and 6.9 g of 1-octyne were heated to reflux for 6 hours within an atmosphere of nitrogen in the presence of 40 mg of cuprous iodide (I), 160 mg of dichlorobistriphenylphosphine palladium(II) and 320 mg of triphenylphosphine. After reaction, the temperature was reduced to the room temperature and the triethyl amine was removed. After the product was extracted with toluene, the toluene layer was washed with 1N-hydrochloric acid and then with water, followed by removing the toluene. The resultant solid was recrystalized twice with hexane to obtain 12.1 g of a compound (a) of the formula:

HEX-#-NAP-$OCH_2$-Ph-H

(3) In 200 ml of ethanol, within hydrogen atmosphere, 12.1 g of the compound (a) were hydrogenated and hydrogenolyzed with use of 0.5 g of 5% palladium carbon. After reaction, the catalyst was filtered off, followed by removing the ethanol. The resulting solid was recrystalized with hexane to obtain 6.3 g of 6-n-octyl-2-naphthol.

(4) Into 60 ml of dry pyridine, were dissolved 4.0 g of 6-n-octyl-2-naphthol, followed by cooling with ice bath to a temperature below 10°C. Thereto were added dropwise 5.7 g of trifluoro-methanesulfonic anhydride at a temperature below 10°C, followed by continuing stirring for 24 hours at the room temperature. Thereafter, the reaction product was thrown into iced water and extracted with toluene. The toluene layer was washed subsquently with 1N-hydrochloric acid, with water, with aqueous sodium hydrogen carbonate and then with water, followed by romoving the toluene to obtain 5.8 g of oily trifluoromethanesulfonate of 6-n-octyl-2-naphthol.

(5) In 30 ml of triethylamine, within nitrogen atmosphere, 1.4 g of trifluoromethanesulfonate of 6-n-octyl-2-naphthol and 1.0 g of p-n-decyloxy-m-fluorophenylacetylene were reacted for 6 hours at reflux temperature in the presence of 16 mg of dichlorobistriphenylphosphine palladium(II), 4 mg of cuprous iodide and 32 mg of triphenylphosphine. After reaction, the triethyl amine was removed, and the product was extracted with toluene. The toluene layer was washed with 1N-hydrochloric acid and then water, and treated with silica gel column, followed by removing the toluene. The resulting solid was recrystalized three times with ethanol to obtain 0.9 g of Compound No.144 of the present invention.

IR ($cm^{-1}$) 2920 2848 1522 1473 1323 1303 1274 1131 1098 1017 893 878 822 480

NMR (ppm) 0.84-0.94(m,6H) 1.20-1.53(m,24H) 1.63-1.77 (m,2H) 1.77-1.89(m,2H) 2.75(t,2H) 4.04(t,2H) 6.91(t,2H) 7.25-7.31(m,2H) 7.36 (dd,1H) 7.52 (dd,1H) 7.59(s,1H) 7.74(d,1H) 7.99(s,1H)

$^{19}$F-NMR(ppm) -58.4(t,1F)

| | | | | |
|---|---|---|---|---|
| Elemental analysis,% | Observed | C: 83.87 | H: 9.31 | F: 3.83 |
| | Theoretical | C: 84.05 | H: 9.14 | F: 3.70 |

Example 10 [Compound No.133]

(1) Example 9 (1)-(4) was repeated except that 1-octyne was substituted with 1-decyne to obtain trifluoromethanesulfonate of 6-n-decyl-2-naphthol.

(2) Example 9 (5) was repeated except that trifluoromethanesulfonate of 6-n-octyl-2-naphthol and p-n-decyloxy-m-fluorophenyl-acetylene were substituted with trifluoromethanesulfonate of 6-n-decyl-2-naphthol and p-n-heptyloxy-m-fluorophenyl-acetylene, respectively, to obtain Compound No.133.

Example 11 [Compound No.136]

(1) Example 9 (1)-(4) was repeated except that 1-octyne was substituted with 1-nonyne to obtain trifluoromethanesulfonate of 6-n-nonyl-2-naphthol.

(2) Example 9 (5) was repeated except that trifluoromethanesulfonate of 6-n-octyl-2-naphthol and p-n-decyloxy-m-fluorophenyl-acetylene were substituted with trifluoromethanesulfonate of 6-n-nonyl-2-naphthol and p-n-octyloxy-m-fluorophenyl-acetylene, respectively, to obtain Compound No.136.

Example 12 [Compound No.141]

Example 11 (2) was repeated except that p-n-octyloxy-m-fluorophenyl-acetylene was substituted with p-n-nonyloxy-m-fluorophenyl-acetylene to obtain Compound No.141.

Example 13 [Compound No.114]

(1) To 70 ml of DMSO containing dissolved therein 8.7 g of 2,3-difluoro-4-iodophenol prepared as in Example 8 (2), was added a solution of 1.4 g of sodium hydroxide dissolved in 5 ml of water, and stirred into a homogeneous solution. Then, 6.9 g of n-decyl bromide were added and stirred for 3 days at room temperature. After reaction, water was added to the product, which was then extracted with hexane. The hexane layer was washed with water, and the hexane was removed to obtain 12.1 g of liquid 4-n-decyloxy-2,3-difluoro-iodobenzene.

(2) In 100 ml of triethylamine, within nitrogen atmosphere, 4.0 g of trifluoromethanesulfonate of 6-n-octyl-2-naphthol and 1.3 g of 3-methyl-1-butyne-3-ol were reacted for 6 hours at reflux temperature in the presence of 36 mg of dichlorobistriphenylphosphine palladium(II), 9 mg of cuprous iodide(I) and 72 mg of triphenylphosphine as catalysts. After reaction, the triethyl amine was removed, and the product was extracted with toluene. The toluene layer was washed with 1N-hydrochloric acid and then water, followed by removing the toluene. Thereafter, the product was dissolved in 300 ml of toluene, and 0.7 g of sodium hydroxide powder was added thereto, followed by refluxing for an hour. After reaction, cooled to the room temperature, the toluene layer was washed with water, and the toluene was removed, followed by recrystalizing the product with methanol to obtain 2.1 g of 6-n-octyl-2-ethynylnaphthalene.

(3) In 30 ml of triethylamine, within nitrogen atmosphere, 1.5 g of 4-n-decyloxy-2,3-difluoroiodobenzene and 1.0 g of 6-n-octyl-2-ethynylnaphthalene were reacted for 3 hours at the room temperature in the presence of 12 mg of dichlorobistriphenylphosphine palladium(II) and 3 mg of cuprous iodide(I) as catalysts. After reaction, the triethyl amine was removed, and the product was extracted with toluene. The toluene layer was washed with 1N-hydrochloric acid and then water, and treated with silica gel column. After removing the toluene, the product was recrystalized three times with ethanol to obtain 0.9 g of Compound No.114 of the present invention.

IR ($cm^{-1}$) 2922 2854 1636 1518 1470 1305 1093 888 818

NMR (ppm) 0.86-0.96(m,6H) 1.20-1.55(m,24H) 1.66-1.79 (m,2H) 1.79-1.91(m,2H) 2.78(t,2H) 4.06(t,2H) 6.69-6.76(m,1H) 7.20-7.27 (m,1H) 7.37(dd,1H) 7.55(dd,1H) 7.60(s,1H) 7.75(d,2H) 8.03(s,1H)

$^{19}F$-NMR(ppm) -57.8(dd,1F) -82.7(dd,1F)

| Elemental analysis,% | | | | |
|---|---|---|---|---|
| | Observed | C: 81.43 | H: 8.47 | F: 6.98 |
| | Theoretical | C: 81.20 | H: 8.65 | N: 7.14 |

Example 14 [Compound No.103]

(1) Example 13 (2) was repeated except that trifluoromethanesulfonate of 6-n-octyl-2-naphthol was substituted with trifluoromethanesulfonate of 6-n-decyl-2-naphthol to obtain 6-n-decyl-2-ethynylnaphthalene.

(2) Example 13 (1) was repeated except that n-decyl bromide was substituted with n-heptyl bromide to obtain 4-n-heptyloxy-2,3-difluoroiodo-benzene.

(3) Example 13 (3) was repeated except that 4-n-decyloxy -2,3-difluoroiodobenzene and 6-n-octyl-2-ethynylnaphthalene were substituted with 4-n-heptyloxy-2,3-difluoroiodobenzene and 6-n-decyl-2-ethynylnaphthalene, respectively, to obtain Compound No.103.

Example 15 [Compound No.106]

(1) Example 13 (3) was repeated except that trifluoromethanesulfonate of 6-n-octyl-2-naphthol was substituted with trifluoromethanesulfonate of 6-n-nonyl-2-naphthol to obtain 6-n-nonyl-2-ethynylnaphthalene.

(2) Example 13 (2) was repeated except that n-decyl bromide was substituted with n-octyl bromide to obtain 4-n-octyloxy-2,3-difluoroiodo-benzene.

(3) Example 13 (4) was repeated except that 4-n-decyloxy -2,3-difluoroiodobenzene and 6-n-octyl-2-ethynylnaphthalene were substituted with 4-n-octyloxy-2,3-difluoroiodobenzene and 6-n-nonyl-2-ethynylnaphthalene, respectively, to obtain Compound No.106.

Example 16 [Compound No.111]

(1) Example 13 (1) was repeated except that n-decyl bromide was substituted with n-nonyl bromide to obtain 4-n-nonyloxy -2,3-difluoroiodo-benzene.

(2) Example 13 (3) was repeated except that 4-n-decyloxy -2,3-difluoroiodobenzene and 6-n-octyl-2-ethynylnaphthalene were substituted with 4-n-nonyloxy-2,3-difluoroiodobenzene and 6-n-nonyl-2-ethynylnaphthalene, respectively, to obtain Compound No.111.

Example 17 [Compound No.402]

(1) To 2.8 g of 6-benzyloxy-2-naphthoic acid, prepared by reacting 6-hydroxy-2-naphthoic acid with benzyl chloride in the presence of a base, were added 10 ml of thionyl chloride, and refluxed for 6 hours. Excess of thionyl chloride was removed with an aspirator to prepare solid 6-benzyloxy-2-naphthoic acid chloride, which was used as such without any purification for the following reaction.

(2) To 6-benzyloxy-2-naphthoic acid chloride, were added 50 ml of dry toluene and 20 ml of dry pyridine, and the mixture was cooled with ice bath to a temperature below 10°C. Thereafter, 0.9 g of oa 2-methylbutanol was added thereto, and stirring was continued for an hour in that condition and then for 5 hours at 80°C, followed by reducing the temperature to the room temperature and then throwing the product into iced water. The toluene layer was washed successively with 1N-hydrochloric acid, with water, with aqueous sodium hydrogen carbonate and then with water, followed by removing the toluene and then recrystalizing the product with ethanol to obtain 2.9 g of white solid 2-methylbutyl 6-benzyloxy-2-naphthoate.

(3) In ethanol, within hydrogen atmosphere, 2.9 g of 2-methylbutyl 6-benzyloxy-2-naphthoate were hydrogenolyzed with use of 0.2 g of 5% palladium carbon. After comfirming that no absorption of hydrogen was observed, the catalyst was filtered off, followed by removing the ethanol. The resulting solid was recystalized with hexane to obtain 1.8 g of oa 2-methylbutyl 6-hydroxy-2-naphthoate.

(4) Into 30 ml of pyridine, were dissolved 1.8 g of oa 2-methylbutyl 6-hydroxy-2-naphthoate, and cooled to a temperature below 10° with ice bath. Thereto were added 2.3 g of TFMSA dropwise, followed by stirring for an hour in that condition and then for 24 hours at the room temperature. After reaction, the product was thrown into iced water and extracted with toluene. The toluene layer was washed with IN-hydrochloric acid, with water, with aqueous sodium hydrogen carbonate and then with water, followed by removing the toluene to obtain 2.6 g of oily trifluoromethane sulfonic acid ester of 2-methylbutyl 6-hydroxy-2-naphthoate.

(5) In ethanol, within hydrogen atmosphere, 20.0 g of 2-bromo-5-nitropyridine were reduced with use of 1.0 g of platinum dioxide. After comfirming that no absorption of hydrogen was observed, the catalyst was filtered off. After removing the ethanol, the product was recystalized with toluene to obtain 17.4 g of 5-amino-2-bromopyridine.

(6) To 900 ml of water, were added 16.0 g of 5-amino-2-bromopyridine and 95 g of 35% hydrochloric acid, and cooled to a temperature below 10°C. Thereto were added dropwise 20 ml of an aqueous solution of 6.4 g of sodium nitrite at a temperature below 10°C, followed by continuing stirring for an hour in that conditions. An aqueous solution of 30 g of potassium iodide was added thereto, and stirred in that condition until no nitrogen was generated. After comfirming that no generation of nitrogen was observed, the product was neutralized with sodium hydroxide, follwed by adding sodium thiosulfate and then continuing stirring for an hour. The resultant precipitates were filtered, dried and then recrystalized with hexane to obtain 20 g of 2-bromo-5-iodopyridine.

(7) In 150 ml of triethylamine, within nitrogen atmosphere, 20 g of 2-bromo-5-iodopyridine and 10.0 g of 1-decyne were reacted for 5 hours at the room temperature in the presence of 240 mg of dichlorobistriphenylphosphine palladium(II) and 60 mg of cuprous iodide(I) as catalysts. After reaction, the triethylamine was removed and the product was extracted with hexane. The hexane layer was washed with 1N-hydrochloric acid and then with water, and the hexane was removed, followed by dissolving in ethanol and hydrogenating the product within hydrogen atmosphere with use of platinumdioxide. After comfirming that no absorption of hydrogen was observed, the catalyst was filtered off, followed by removing the ethanol. The resulting solid was dissolved in hexane to remove impurities and then the hexane was removed to obtain 16.6 g of liquid 2-bromo-5-n-decylpyridine.

(8) In 150 ml of triethylamine, within nitrogen atmosphere, 16.6 g of 2-bromo-5-n-decylpyridine and 5.6 g of 3-methyl-1-butyne-3-ol were reacted for 6 hours at reflux temperature in the presence of 200 mg of dichlorobistriphenylphosphine palladium(II), 50 mg of cuprous iodide(I) and 400 mg of triphenylphosphine. After reaction, the triethylamine was removed and the product was extracted with hexane. The hexane layer was washed with 1N-hydrochloric acid and then with water, and the hexane was removed, followed by dissolving the product in anhydrous toluene, adding thereto sodium hydroxide powder and refluxing for an hour. Thereafter, the toluene layer was cooled to the room temperature and washed with water, followed by removing the toluene. The product was dissolved in methanol and treated with activated carbon, and then methanol was removed to obtain 6.3 g of liquid 2-ethynyl-5-n-decylpyridine.

(9) In 30 ml of triethylamine, within nitrogen atmosphere, 2.4 g of trifluoromethane sulfonic acid ester of 2-methylbutyl 6-hydroxy-2-naphthoate and 1.8 g of 2-ethynyl -5-n-decylpyridine were reacted for 6 hours at reflux temperature in the presence of 20 mg of dichlorobistriphenylphosphine palladium(II), 5 mg of cuprous iodide(I) and 40 mg of triphenylphosphine. After reaction, the triethylamine was removed and the product was extracted with toluene. The toluene layer was washed with 1N-hydrochloric acid and then with water,

and treated with silica gel column, followed by removing the hexane. The resulting solid was recrystalized thrice with ethanol to obtain 1.3 g of Compound No.402 of the present invention.

IR ($cm^{-1}$) 2920 2852 1715 1468 1280 1228 1185 824 748 474

NMR (ppm) 0.85(t,3H) 0.96(t,3H) 1.03(d,3H) 1.16-1.38 (m,16H) 1.50-1.70(m,2H) 1.81-1.99(m,1H) 2.61 (t,2H) 4.14-4.30(m,2H) 7.48-7.52(m,2H) 7.67 (dd,1H) 7.84(d,1H) 7.92(d,1H) 8.07(dd,1H) 8.11(s,1H) 8.47(s,1H) 8.57(s,1H)

| | | | | |
|---|---|---|---|---|
| Elemental analysis,% | Observed | C: 81.83 | H: 8.56 | N: 3.13 |
| | Theoretical | C: 81.99 | H: 8.49 | N: 2.90 |

Example 18 [Compound No.211]

(1) To an aqueous solution (acetic acid 40 ml/water 8 ml/conc.sulfuric acid 1.2 ml) containing dissolved therein 13.8 g of 1,2-difluoro-3-n-nonyloxybenzene prepared by reacting n-nonyl bromide with alkali metal 2,3-difluorophenolate, were added 2.45 g of periodic acid and 5.46 g of iodine, and reacted for 6 hours at 70°C. After reaction, the product was cooled to the room temperature and thrown into 200 ml of iced water, followed by extracting the resulting oil with hexane. The hexane layer was washed successively with 1N-sodium hydroxide aqueous solution, with 1N-sodium thiosulfate aqueous solution and water, followed by removing the hexane to obtain 18.3 g of 2,3-difluoro-4-n-nonyloxy-iodobenzene.

(2) In 150 ml of triethylamine, within nitrogen atmosphere, 18.3 g of 2,3-difluoro-4-n-nonyloxy-iodobenzene and 4.8 g of 3-methyl-1-butyne-3-ol were reacted for 6 hours at the room temperature in the presence of 100 mg of dichlorobistriphenylphosphine palladium(II) and 25 mg of cuprous iodide (I) as catalysts to obtain 13.8 g of a compound (b) of the formula : NON-O-FPhF-#-C$(Me)_2$OH.

(3) To a solution of 13.8 g of the compound (b) dissolved in 500 ml of anhydrous toluene, 3.3 g of potassium hydroxide powder were added, and heated for an hour to the reflux temperature. After cooling to the room temperature, the toluene layer was washed with water, followed by removing the toluene to obtain 8.0 g of oily 2,3-difluoro-4-n-nonyloxyphenylacetylene.

(4) In 60 ml of triethylamine, within nitrogen atmosphere, 1.6 g of 2,3-difluoro-4-n-nonyloxyphenylacetylene and 2.0 g of 6-bromo-2-n-nonyloxynaphthalene prepared by reacting n-nonyl bromide with alkali metal 6-bromo-2-naphthol were reacted for 8 hours at the reflux temperature in the presence of 20 mg of dichlorobistriphenylphosphine palladium(II), 5 mg of cuprous iodide (I) and 40 mg of triphenylphosphine as catalysts. Thereafter, the triethylamine was removed, and the product was extracted with toluene. The toluene layer was washed with IN-hydrochloric acid and then with water, and treated with silica gel column, followed by recrystalizing the product three times with ethanol to obtain 1.7 g of white crystalline Compound No.211 of the present invention.

| | | | | |
|---|---|---|---|---|
| Elemental analysis,% | Observed | C: 78.73 | H: 8.45 | F: 7.12 |
| | Theoretical | C: 78.84 | H: 8.39 | F: 6.93 |

Example 19 [Compound No.237]

(1) Example 18 (1)-(3) were repeated except that 1,2-difluoro-3-(7-octenyloxy)benzene prepared by reacting 8-bromo-1-octene with alkali metal 2,3-difluorophenolate was used instead of 1,2-difluoro-3-n-nonyloxybenzene in Example 18 (1) to obtain 2,3-difluoro-4-(7-octenyloxy)phenylacetylene.

(2) In 60 ml of triethylamine, within nitrogen atmosphere, 2.0 g of 2,3-difluoro-4-(7-octenyloxy)phenylacetylene and 2.7 g of 6-bromo-2-n-decyloxynaphthalene prepared by reacting n-decyl bromide with alkali metal 6-bromo-2-naphthol were reacted for 8 hours at the reflux temperature in the presence of 28 mg of dichlorobistriphenylphosphine palladium(II), 7 mg of cuprous iodide (I) and 56 mg of triphenylphosphine as catalysts. Then, the triethylamine was removed, and the product was extracted with toluene. The toluene laver was washed with 1N-hydrochloric acid and then with water, and treated with silica gel column, followed by recrystalizing the product three times with ethanol to obtain 1.6 g of white crystalline Compound No.237 of the present invention.

| Elemental analysis,% | | | | |
|---|---|---|---|---|
| | Observed | C: 79.37 | H: 7.83 | F: 7.13 |
| | Theoretical | C: 79.12 | H: 8.06 | F: 6.96 |

Example 20 [Compound No.222]

(1) Example 18 (1)-(3) were repeated except that 1,2-difluoro-3-(n-octyloxy)benzene prepared by reacting n-octylbromide with alkali metal 2,3-difluorophenolate was used instead of 1,2-difluoro-3-n-nonyloxybenzene in Example 18 (1) to obtain 2,3-difluoro-4-(n-octyloxy)phenylacetylene.
(2) Example 18 (4) was repeated except that 1.5 g of 2,3-difluoro-4-(n-octyloxy)phenyl-acetylene and 2.0 g of 6-bromo-2-(9-decenyloxy)naphthalene prepared by reacting 9-decene-1-ol in the presence of DEAD and triphenylphosphine were used instead of 2,3-difluoro-4-n-nonyloxyphenylacetylene and 6-bromo-2-n-nonyloxynaphthalene, respectively, to obtain 1.7 g of white crystalline Compound No.222 of the present invention.

| Elemental analysis,% | | | | |
|---|---|---|---|---|
| | Observed | C: 78.83 | H: 7.95 | F: 7.21 |
| | Theoretical | C: 78.95 | H: 7.89 | F: 7.14 |

Example 21 [Compound No.62]

(1) To 400 ml of dry ethanol, were added 25.0 g of 6-bromo-2-naphthol and 7.5 g of potassium hydroxide, and stirred to obtain a homogeneous solution. Then 27.4 g of oa 2-methylbutyl p-toluenesulfonate were added thereto and heated to reflux for 20 hours. After reaction, the ethanol was removed, and then water was added. The resulting solid was filterred and recrystalized with methanol to obtain 24.6 g of oa 6-bromo-2-(2′-methylbutyloxy)naphthalene.
(2) In 60 ml of triethylamine, within nitrogen atmosphere, 2.0 g of oa 6-bromo-2-(2′-methylbutyloxy)naphthalene and 1.7 g of 2-ethynyl-5-n-decylpyridine as prepared in Example 17 (8) were reacted for 8 hours at reflux temperature in the presence of 24 mg of dichlorobistriphenylphosphine palladium(II), 6 mg of cuprous iodide (I) and 48 mg of triphenylphosphine. After reaction, the triethylamine was removed and the product was extracted with toluene. The toluene layer was washed with 1N-hydrochloric acid and then with water, and treated with silica gel column, followed by removing the toluene. The resulting solid was recrystalized thrice with ethanol to obtain 1.8 g of Compound No.62 of the present invention.

| Elemental analysis,% | | | | |
|---|---|---|---|---|
| | Observed | C: 84.11 | H: 9.23 | N: 3.00 |
| | Theoretical | C: 84.39 | H: 9.01 | N: 3.08 |

Example 22 [Compound No.256]

(1) To 100 ml of dry N,N-dimethylformamide (DMF) containing 1.6 g of sodium hydride, were added dropwise 10.0 g of n-decyl alcohol at a temperature below 50°C in such a speed not causing vigorous evolution of hydrogen. After confirming that no evolution of hydrogen was observed, the DMF solution was cooled with ice bath to a temperature below 10°C, and 15.0 g of 2,5-dibromopyridine were added thereto, followed by continuing stirring for 1 hour in that conditions and then for 2 hours at 80°C. After cooling to the room temperature, the product was thrown into iced water, and the formed oil was extracted with hexane. The hexane phase was washed with water, followed by removing the hexane to obtain 18.9 g of liquid 5-bromo-2-decyloxypyridine.
(2) In 80 ml of triethylamine, within nitrogen atmosphere, 5.0 g of 5-bromo-2-decyloxypyridine and 1.6 g of 3-methyl-1-butyne-3-ol were reacted for 8 hours at reflux temperature in the presence of 56 mg of dichlorobistriphenylphosphine palladium(II), 14 mg of cuprous iodide(I) and 112 mg of triphenylphosphine. After reaction and cooling to the room temperature, the triethylamine was removed and the product was

extracted with toluene. The toluene layer was washed with 1N-hydrochloric acid and then with water, and the toluene was removed. Then, the product was dissolved in methanol and treated with active carbon, followed by removing the methanol. To the resulting liquid, were added 150 ml dry toluene and 1.3 g of powdery sodium hydroxide, and heated for an hour to reflux temperature. After cooling, the toluene layer was washed with water, followed by removing the toluene to obtain 2.3 g of oily 5-ethynyl-2-n-decyloxypyridine.

(3) In 70 ml of triethylamine, within nitrogen atmosphere, 2.3 g of 5-ethynyl-2-n-decyloxypyridine and 2.6 g of oa 6-bromo-2-(2′-methylbutyloxy)naphthalene as prepared in Example 21 (1) were reacted for 8 hours at reflux temperature in the presence of 32 mg of dichlorobistriphenylphosphine palladium(II), 8 mg of cuprous iodide (I) and 64 mg of triphenylphosphine. After reaction and cooling to the room temperature, the triethylamine was removed and the product was extracted with toluene. The toluene layer was washed with 1N-hydrochloric acid and then with water, and treated with silica gel column, followed by removing the toluene. The resulting solid was recrystalized thrice with ethanol to obtain 2.2 g of white crystalline Compound No.256 of the present invention.

| | | | | |
|---|---|---|---|---|
| Elemental analysis,% | Observed | C: 81.73 | H: 8.55 | N: 3.04 |
| | Theoretical | C: 81.54 | H: 8.70 | N: 2.97 |

Example 23 [Compound No.247]

(1) Example 18 (1) was repeated except that 14.5 g of 1,2-difluoro-3-n-decyloxybenzene prepared by reacting n-decyl bromide with alkali metal 2,3-difluorophenolate were used instead of 13.8 g of 1,2-difluoro-3-n-nonyloxybenzene to obtain 19.4 g of 2,3-difluoro-4-n-decyloxy-iodobenzene.

(2) In 150 ml of triethylamine, within nitrogen atmosphere, 19.4 g of 2,3-difluoro-4-n-nonyloxy-iodobenzene and 4.9 g of 3-methyl-1-butyne-3-ol were reacted for 8 hours at the room temperature in the presence of 100 mg of dichlorobistriphenylphosphine palladium(II) and 25 mg of cuprous iodide (I) as catalysts, followed by removing the triethylamine and extracting the product with toluene. The toluene layer was washed with 1N-hydrochloric acid and then with water, and the toluene was removed. Then, the product was dissolved in methanol and treated with active carbon, followed by removing the methanol. To the resulting liquid, were added 450 ml dry toluene and 3.9 g of powdery sodium hydroxide, and heated for an hour to reflux temperature. After cooling, the toluene layer was washed with water, and the toluene was removed to obtain 9.4 g of oily 2,3-difluoro-4-n-decyloxyphenyl-acetylene.

(3) In 60 ml of triethylamine, within nitrogen atmosphere, 2.0 g of 2,3-difluoro-4-n-decyloxyphenylacetylene and 2.0 g of oa 6-bromo-2-(2-methylbutyloxy)naphthalene as prepared in Example 21 (1) were reacted for 8 hours at the reflux temperature in the presence of 24 mg of dichlorobistriphenylphosphine palladium(II), 6 mg of cuprous iodide (I) and 48 mg of triphenylphosphine as catalysts. After reaction and cooling to the room temperature, the triethylamine was removed, and the product was extracted with toluene. The toluene layer was washed with 1N-hydrochloric acid and then with water, and treated with silica gel column, followed by recrystalizing the product three times with ethanol to obtain 2.0 g of white crystalline Compound No.247 of the present invention.

| | | | | |
|---|---|---|---|---|
| Elemental analysis,% | Observed | C: 78.41 | H: 7.73 | F: 7.65 |
| | Theoretical | C: 78.26 | H: 7.91 | F: 7.51 |

Example 24 [Compound No.67]

(1) Into 200 ml of anhydrous tetrahydrofurane (THF), were dissolved 15.2 g of o-difluorobenzene, and cooled with dry ice-acetone bath to a temperature below -60°C. Then, 90 ml of 15% solution of n-butyl lithium in hexane were added dropwise thereto at a temperature below -60°C, and stirring was continued for 3 hours in that conditions. Subsequently, 25 ml of n-nonyl aldehyde were added dropwise at a temperature below -60°C, and stirring was continued for 30 minutes in that conditions. Thereafter, the dry ice-acetone bath was removed, and then stirring was continued until the temperature reached 0°C. Water and

then 1N-hydrochloric acid were added, and subsequently the product was extracted with diethylether, followed by washing the ether phase with water and removing the ether to obtain 35.5 g of a liquid compound (c) of the formula :

DEC-CH(OH)-FPhF-H.

(2) To 1 l of toluene, 35.5 g of the compound (c) and 4.0 g of p-toluenesulfonic acid were added, and heated for 3 days to reflux with removing formed water. Thereafter, toluene phase was washed with water, followed by removing the toluene and then distiling under reduced pressure to obtain 17.6 g of a liquid compound (d) of the formula:

HEP-CH=CH-FPhF-H.

(3) Into 300 ml of ethanol, were dissolved 17.6 g of the compound (d), followed by carrying out hydrogenation using 2.0 g of 5% palladium carbon. After confirming that no absorption of hydrogen was observed, the catalyst was filtered off, and then the ethanol was removed to obtain 17.5 g of liquid 1-n-nonyl-2,3-difluorobenzene.

(4) Into 120 ml of anhydrous THF, were dissolved 17.5 g of 1-n-nonyl-2,3-difluorobenzene, and cooled with dry ice-acetone bath to a temperature below -60°C. Then, 57 ml of 15% solution of n-butyl lithium in hexane were added dropwise thereto at a temperature below -60°C and stirring was continued for 3 hours in that conditions, followed by adding 22.2 g of iodine dropwise thereto at a temperature below -60°C and then stirring for 30 minutes in that conditions. Thereafter, the dry ice-acetone bath was removed, and then stirring was continued until the temperature reached 0°C. The reaction product was thrown into 100 ml of water, and the resulting oil was extracted with hexane. The hexane layer was washed successively with 1N-sodium hydroxide aqueous solution, with 1N-sodium thio-sulfate aqueous solution and with water, followed by removing the hexane to obtain 26.8 g of 2,3-difluoro-4-n-nonyl-iodobenzene.

(5) In 150 ml of triethylamine, within nitrogen atmosphere, 15.0 g of 2,3-difluoro-4-n-nonyl-iodobenzene and 4.1 g of 3-methyl-1-butyne-3-ol were reacted for 8 hours at the room temperature in the presence of 88 mg of dichlorobistriphenylphosphine palladium(II) and 22 mg of cuprous iodide (I) as catalysts, followed by removing the triethylamine. The product was extracted with toluene. The toluene layer was washed with 1N-hydrochloric acid and then with water, and the toluene was removed. Then, the product was dissolved in methanol and treated with active carbon, followed by removing the methanol. To the resulting liquid, were added 400 ml dry toluene and 3.3 g of powdery sodium hydroxide, and heated for an hour to reflux temperature. After cooling, the toluene layer was washed with water, followed by removing the toluene to obtain 6.4 g of oily 2,3-difluoro-4-nonylphenylacetylene.

(6) In 60 ml of triethylamine, within nitrogen atmosphere, 2.0 g of 2,3-difluoro-4-nonylphenylacetylene and 2.5 g of oa 6-bromo-2-(1-methylheptyloxy)naphthalene were reacted for 8 hours at reflux temperature in the presence of 7 mg of cuprous iodide (I), 28 mg of dichlorobistriphenylphosphine palladium(II) and 56 mg of triphenylphosphine. After reaction and cooling to the room temperature, the triethyl amine was removed and the product was extracted with toluene. The toluene phase was washed with 1N-hydrochloric acid and then with water, purified with silica gel colmun, and recrystalized thrice with ethanol to obtain 1.6 g of white crystalline Compound No.67 of the present invention.

| | | | | |
|---|---|---|---|---|
| Elemental | Observed | C: 80.93 | H: 8.61 | F: 7.52 |
| analysis,% | Theoretical | C: 81.08 | H: 8.49 | F: 7.34 |

Example 25 [Compound No.252]

(1) In 200 ml of triethylamine, within nitrogen atmosphere, 17.0 g of 6-bromo-2-n-decyloxynaphthalene and 4.9 g of 3-methyl-1-butyne-3-ol were reacted for 8 hours at reflux temperature in the presence of 164 mg of dichlorobistriphenylphosphine palladium(II), 41 mg of cuprous iodide (I) and 328 mg of triphenylphosphine, followed by cooling to the room temperature and removing the triethylamine. The product was extracted with toluene. The toluene layer was washed with 1N-hydrochloric acid and then with water, and the toluene was removed. Then, the product was dissolved in methanol and treated with active carbon, followed by removing the methanol. To the resulting solid, were added 400 ml dry toluene and 3.7 g of powdery sodium hydroxide, and heated for an hour to reflux temperature. After cooling, the toluene layer was washed with water, followed by removing the toluene. The resulting solid was recrystalized with methanol to obtain 7.8 g of 2-n-decyloxy-6-ethynylnaphthalene.

(2) In 100 ml of triethylamine, within nitrogen atmosphere, 5.0 g of 2-n-decyloxy-6-ethynylnaphthalene and

3.9 g of 1,2-difluoro-3-iodobenzene prepared by reacting o-difluorobenzene with n-butyl lithium and then with iodine were reacted for 8 hours at the room temperature in the presence of 9 mg of cuprous iodide (I) and 36 mg of dichlorobistriphenylphosphine palladium(II) as catalyst. After reaction, the triethyl amine was removed and the product was extracted with toluene. The toluene phase was washed with 1N-hydrochloric acid and then with water, and recrystalized with ethanol to obtain 5.6 g of a compound (e) of the formula : DEC-O-NAP-#-FPhF-H

(3) Into 100 ml of anhydrous THF, were dissolved 4.7 g of the compound (e), and cooled with dry ice-acetone bath to a temperature below -60°C. Then, 8 ml of 15% solution of n-butyl lithium in hexane were added dropwise thereto at a temperature below -60°C, and stirring was continued for 3 hours in that conditions, followed by adding 1.9 g of trimethyl borate thereto at a temperature below -60°C and stirring was continued for 30 minutes in that conditions. Thereafter, the dry ice-acetone bath was removed, and then stirring was continued until the temperature reached 0°C. Water and then 1N-hydrochloric acid were added, and subsequently the product was extracted with diethylether, followed by washing the ether phase with water and removing the ether to obtain 4.8 g of white solid compound (f) of the formula :

DEC-O-NAP-#-FPhF-B$(OH)_2$.

(4) Into diethyl ether solution containing the compound (f) dissolved therein, were added 25 ml of 10% aqueous hydrogen peroxide, and stirred for 1 hour at the room temperature and then for 2 hours at the reflux temperature. Thereafter, the ether phase was washed with water, and then the ether was removed to obtain 4.0 g of compound (g) of the formula : DEC-O-NAP-#-FPhF-OH.

(5) Into 100 ml of anhydrous THF, were dissolved 2.5 g of the compound (g), 1.8 g of triphenylphosphine and 0.9 g of oa 2-octanol, and the solution was cooled with ice bath to a temperature below 10°C. To this solution, was added dropwise 1.2 g of diethyl azodiformate at a temperature below 10°C, and stirring was continued for 1 hour in that conditions and then for 24 hours at the room temperature, followed by removing the soluvent. Hexane-soluble matter was dissolved in toluene and purified with silica gel column and then recrystalized thrice with ethanol to obtain 1.2 g of white solid Compound No.252 of the present invention.

| | | | | |
|---|---|---|---|---|
| Elemental | Observed | C: 78.67 | H: 8.55 | F: 7.01 |
| analysis,% | Theoretical | C: 78.84 | H: 8.39 | F: 6.93 |

Example 26 [Compound No.168]

(1) In 100 ml of triethylamine, within nitrogen atmosphere, 5.3 g of 1-decyne and 10.0 g of 6-bromo-2-benzyloxynaphthalene prepared by reacting benzyl chloride with alkali metal 6-bromo-2-naphtholate were reacted for 8 hours at reflux temperature in the presence of 28 mg of cuprous iodide(I), 112 mg of dichlorobistriphenylphosphine palladium(II) and 224 mg of triphenyl-phosphine. After reaction, the triethylamine was removed, and the product was extracted with toluene. The toluene phase was washed with 1N-hydrochloric acid and then with water, followed by removing the toluene. The product was recrystalized with ethanol to obtain 10.0 g of a compound (h) of the formula: OCT-#-NAP-$OCH_2$-Ph-H.

(2) Within an atmosphere of hydrogen, in 150 ml of ethanol, 10.0 g of the compound (h) was hydrogenized and hydrogenolyzed using 0.5 g of 5% palladium carbon. After confirming that no absorption of hydrogen was observed, the catalyst was filtered off, followed by removing the ethanol and then recrystalizing the resulting solid with hexane to obtain 6.5 g of 6-n-decyl-2-hydroxynaphthalene.

(3) Into 100 ml of anhydrous pyridine, were dissolved 6.5 g of 6-n-decyl-2-hydroxynaphthalene, and cooled to a temperature below 10°C. Then, 7.1 g of TFMSA was added dropwise at a temperature below 10°C, followed by stirring for 24 hours at the room temperature. The resulting solution was thrown into iced water and acidified with hydrochloric acid, and then extracted with hexane. The hexane phase was washed with water and the hexane was removed to obtain an oily compound (i) of the formula :

DEC-NAP-O-$SO_2CF_3$.

(4) In 70 ml of triethylamine, within nitrogen atmosphere, 5.0 g of the compound (i) and 1.2 g of 3-methyl-1-butyne-3-ol were reacted for 8 hours at reflux temperature in the presence of 11 mg of cuprous iodide (I), 44 mg of dichlorobistriphenylphosphine palladium(II) and 88 mg of triphenylphosphine. After reaction and cooling to the room temperature, the triethyl amine was removed and the product was extracted with toluene. The toluene phase was washed with 1N-hydrochloric acid and then with water, followed by removing the toluene. Then, the product was dissolved in methanol and treated with active carbon, followed by removing the methanol. To the resulting liquid, were added 200 ml dry toluene and 1.0 g of powdery sodium

hydroxide, and heated for an hour to reflux temperature. After cooling, the toluene layer was washed with water, followed by removing the toluene to obtain 2.4 g of oily 6-n-decyl-2-ethynylnaphthalene.
(5) Example 25 (2)-(5) were repeated except that 6-n-decyl-2-ethynylnaphthalene was used instead of 2-n-decyloxy-6-ethynylnaphthalene to obtain 0.9 g of Compound No.168 of the present invention.

| Elemental analysis,% | | | | |
|---|---|---|---|---|
| | Observed | C: 81.42 | H: 8.49 | F: 7.36 |
| | Theoretical | C: 81.20 | H: 8.65 | F: 7.14 |

Example 27 [Compound No.255]

(1) In 200 ml of triethylamine, within nitrogen atmosphere, 15.0 g of 4-n-decyloxy-3-fluorobromobenzene and 4.6 g of 3-methyl-1-butyne-3-ol were reacted for 8 hours at reflux temperature in the presence of 40 mg of cuprous iodide(I), 160 mg of dichlorobistriphenylphosphine palladium(II) and 320 mg of triphenylphosphine. After reaction and cooling to the room temperature, the triethyl amine was removed and the product was extracted with toluene. The toluene phase was washed with 1N-hydrochloric acid and then with water, followed by removing the toluene. Then, the product was dissolved in methanol and treated with active carbon, followed by removing the methanol. To the resulting liquid, were added 400 ml dry toluene and 3.6 g of powdery sodium hydroxide, and heated for an hour to reflux temperature. After cooling, the toluene layer was washed with water, followed by removing the toluene to obtain 7.9 g of oily 4-n-decyloxy-3-fluorophenylacetylene.
(2) In 60 ml of triethylamine, within nitrogen atmosphere, 2.0 g of 4-n-decyloxy-3-fluorophenylacetylene and 2.1 g of oa 6-bromo-2-(2-methylbutyloxy)naphthalene were reacted for 8 hours at reflux temperature in the presence of 6 mg of cuprous iodide(I), 24 mg of dichlorobistriphenylphosphine palladium(II) and 48 mg of triphenylphosphine. After reaction and cooling to the room temperature, the triethyl amine was removed and the product was extracted with toluene. The toluene phase was washed with 1N-hydrochloric acid and then with water, purified with silica gel column and then recrystalized thrice with ethanol to obtain 1.9 g of white crystalline Compound No.255 of the present invention.

| Elemental analysis,% | | | | |
|---|---|---|---|---|
| | Observed | C: 81.29 | H: 8.21 | F: 4.01 |
| | Theoretical | C: 81.15 | H: 8.40 | F: 3.89 |

Example 28 [Compound No.163]

In 60 ml of triethylamine, within nitrogen atmosphere, 2.0 g of 6-n-decyl-2-ethynylnaphthalene and 2.1 g of oa 4-(1-methylheptyloxy)-3-fluorobromobenzene were reacted for 8 hours at reflux temperature in the presence of 6 mg of cuprous iodide(I), 24 mg of dichlorobistriphenylphosphine palladium(II) and 48 mg of triphenylphosphine. After reaction and cooling to the room temperature, the triethyl amine was removed and the product was extracted with toluene. The toluene phase was washed with 1N-hydrochloric acid and then with water, purified with silica gel column and then recrystalized thrice with ethanol to obtain 1.8 g of white crystalline Compound No.163 of the present invention.

| Elemental analysis,% | | | | |
|---|---|---|---|---|
| | Observed | C: 83.91 | H: 9.05 | F: 3.93 |
| | Theoretical | C: 84.05 | H: 9.14 | F: 3.70 |

Example 29 [Compound No.401]

Example 17 (2)-(9) were repeated except that oa 4-methylhexanol was used instead of oa 2-methylbutanol in Example 17 (2) to obtain Compound No.401 of the present invention.

| | | | | |
|---|---|---|---|---|
| Elemental analysis,% | Observed | C: 82.33 | H: 8.67 | N: 2.84 |
| | Theoretical | C: 82.19 | H: 8.81 | N: 2.74 |

Example 30 [Compound No.403]

Example 17 (2)-(9) were repeated except that oa 1-methyloctanol was used instead of oa 2-methylbutanol in Example 17 (2) to obtain Compound No.403 of the present invention.

| | | | | |
|---|---|---|---|---|
| Elemental analysis,% | Observed | C: 82.05 | H: 9.11 | N: 2.55 |
| | Theoretical | C: 82.28 | H: 8.95 | N: 2.67 |

Example 31 [Compound No.314]

In 60 ml of triethylamine, within nitrogen atmosphere, 1.5 g of oa trifluoromethanesulfonate of 6-hydroxy-2-(2-methylbutyl)-naphthoate and 1.0 g of 5-ethynyl-2-n-decyloxypyridine as prepared in Example 22 (2) were reacted for 8 hours at reflux temperature in the presence of 16 mg of dichlorobis-triphenylphosphine palladium(II), 4 mg of cuprous iodide (I) and 32 mg of triphenylphosphine. After reaction and cooling to the room temperature, the triethylamine was removed and the product was extracted with toluene. The toluene layer was washed with 1N-hydrochloric acid and then with water, and treated with silica gel column, followed by removing the toluene. The resulting solid was recrystalized thrice with ethanol to obtain 1.0 g of white crystalline Compound No.314 of the present invention.

| | | | | |
|---|---|---|---|---|
| Elemental analysis,% | Observed | C: 79.13 | H: 8.30 | N: 2.65 |
| | Theoretical | C: 79.36 | H: 8.22 | N: 2.80 |

Example 32 [Compound No.315]

Example 31 was repeated except that oa trifluoromethanesulfonate of 6-hydroxy-2-(1-methylheptyl)naphthoate was used instead of trifluoromethanesulfonate of 2-methylbutyl 6-hydroxy-2-naphthoate to obtain Compound No.315 of the present invention.

| | | | | |
|---|---|---|---|---|
| Elemental analysis,% | Observed | C: 79.80 | H: 8.87 | N: 2.75 |
| | Theoretical | C: 79.85 | H: 8.69 | N: 2.59 |

Example 33 [Compound No.302]

In 60 ml of triethylamine, within nitrogen atmosphere, 2.0 g of oa trifluoromethanesulfonate of 6-hydroxy-2-(2-methylbutyl)naphthoate and 1.5 g of 2,3-difluoro-4-n-decyloxyphenylacetylene as prepared in Example 23 (2) were reacted for 8 hours at reflux temperature in the presence of 20 mg of dichlorobis-triphenylphosphine palladium(II), 5 mg of cuprous iodide (I) and 40 mg of triphenylphosphine. After reaction and cooling to the room temperature, the triethylamine was removed, followed by extracting the product with toluene. The toluene layer was washed with 1N-hydrochloric acid and then with water, purified with silica gel column, and then recrystalized thrice with ethanol to obtain 1.3 g of white crystalline Compound No.302 of the present invention.

| Elemental analysis,% | | | | |
|---|---|---|---|---|
| | Observed | C: 76.33 | H: 7.57 | F: 7.24 |
| | Theoretical | C: 76.40 | H: 7.49 | F: 7.12 |

Example 34 [Compound No.303]

Example 33 was repeated except that oa trifluoromethanesulfonate of 6-hydroxy-2-(1-methylheptyl)naphthoate was used instead of trifluoromethanesulfonate of 2-methylbutyl 6-hydroxy-2-naphthoate to obtain Compound No.303 of the present invention.

| Elemental analysis,% | | | | |
|---|---|---|---|---|
| | Observed | C: 77.21 | H: 7.76 | F: 6.65 |
| | Theoretical | C: 77.08 | H: 7.99 | F: 6.60 |

Example 35 [Compound No.409]

(1) Into 100 ml of anhydrous THF, were dissolved 8.8 g of 1-n-decyl-2,3-difluorobenzene, and cooled with dry ice-acetone bath to a temperature below -60°C. Then, 24 ml of 15% solution of n-butyl lithium in hexane were added dropwise thereto at a temperature below -60°C and stirring was continued for 3 hours in that conditions, followed by adding 9.2 g of iodine dropwise thereto at a temperature below -60°C and then stirring for 30 minutes in that conditions. Thereafter, the dry ice-acetone bath was removed, and then stirring was continued until the temperature reached 0°C. The reaction product was thrown into 300 ml of water, and the resulting oil was extracted with hexane. The hexane layer was washed successively with 1N-sodium hydroxide aqueous solution, with 1N-sodium thio-sulfate aqueous solution and with water, followed by removing the hexane to obtain 12.5 g of 2,3-difluoro-4-n-decyl-iodobenzene.
(2) In 120 ml of triethylamine, within nitrogen atmosphere, 12.5 g of 2,3-difluoro-4-n-decyl-iodobenzene and 3.3 g of 3-methyl-1-butyne-3-ol were reacted for 8 hours at the room temperature in the presence of 72 mg of dichlorobistriphenylphosphine palladium(II) and 18 mg of cuprous iodide (I) as catalysts, followed by removing the triethylamine. The product was extracted with toluene. The toluene layer was washed with 1N-hydrochloric acid and then with water, and the toluene was removed. Then, the product was dissolved in methanol and treated with active carbon, followed by removing the methanol. To the resulting liquid, were added 350 ml dry toluene and 2.6 g of powdery sodium hydroxide, and heated for an hour to reflux temperature. After cooling, the toluene layer was washed with water, followed by removing the toluene to obtain 4.9 g of oily 2,3-difluoro-4-decylphenylacetylene.
(3) In 60 ml of triethylamine, within nitrogen atmosphere, 1.5 g of 2,3-difluoro-4-decylphenylacetylene and 2.3 g of oa trifluoromethanesulfonate of 6-hydroxy-2-(1-methylheptyl)naphthoate were reacted for 8 hours at reflux temperature in the presence of 7 mg of cuprous iodide (I), 28 mg of dichlorobistriphenylphosphine palladium(II) and 56 mg of triphenylphosphine. After reaction, the triethyl amine was removed and the product was extracted with toluene. The toluene phase was washed with 1N-hydrochloric acid and then with water, purified with silica gel colmun, and recrystalized thrice with ethanol to obtain 1.6 g of white crystalline Compound No.409 of this invention.

| Elemental analysis,% | | | | |
|---|---|---|---|---|
| | Observed | C: 79.45 | H: 8.07 | F: 6.85 |
| | Theoretical | C: 79.29 | H: 8.21 | F: 6.79 |

Example 36 [Compound No.609]

(1) Into 100 ml of anhydrous THF, were dissolved 7.0 g of N,N,N',N'-tetramethylmethylenediamine and 8.0 g of the compound (e) as prepared in Example 25 (2), and cooled with dry ice-acetone bath to a temperature below -60°C. Then, 14 ml of 15% solution of n-butyl lithium in hexane were added dropwise thereto at a temperature below -60°C, and stirring was continued for 3 hours in that conditions, followed by introducing

carbon dioxide at a temperature below -60°C. Thereafter, stirring was continued without dry ice-acetone bath until the temperature reached 0°C. Water and then 1N-hydrochloric acid were added, and subsequently the product was extracted with diethylether, followed by washing the ether phase with water and removing the ether to obtain 6.7 g of a compund (j) of the formula : DEC-O-NAP-#-FPhF-COOH.

(2) Into 50 ml of anhydrous THF, were dissolved 2.0 g of the compund (j), 1.2 g of triphenylphosphine and 0.6 g of oa 1-methyl-octanol, and cooled with ice bath to a temperature below 10°C. To this solution, was added dropwise 0.9 g of diethyl azodicarboxylate at a temperature below 10°C, and stirring was continued for 1 hour in that conditions and then for 24 hours at the room temperature, followed by removing the solvent. Hexane-soluble matter was dissolved in toluene and purified with silica gel column, and then recrystalized thrice with ethanol to obtain 1.3 g of white crystalline Compound No.609 of the present invention.

| | | | | |
|---|---|---|---|---|
| Elemental analysis,% | Observed | C: 77.21 | H: 8.12 | F: 6.33 |
| | Theoretical | C: 77.08 | H: 7.99 | F: 6.60 |

Example 37 [Compound No.509]

Example 25 (1)-(2) and then Example 36 (1)-(2) were repeated except that 6-n-decyl-2-hydroxynaphthalene trifluoromethanesulfonate was used instead of 6-bromo-2-n-decyloxynaphthalene in Example 25 (1) to obtain Compound No.509 of the present invention.

| | | | | |
|---|---|---|---|---|
| Elemental analysis,% | Observed | C: 79.11 | H: 8.43 | F: 6.81 |
| | Theoretical | C: 79.29 | H: 8.21 | F: 6.79 |

Example 38 [Compound No.308]

In 60 ml of triethylamine, within nitrogen atmosphere, 2.1 g of oa trifluoromethanesulfonate of 6-hydroxy-2-(2-methylbutyl)naphthoate and 1.5 g of 4-n-decyloxy-3-fluorophenylacetylene as prepared in Example 27 (2) were reacted for 8 hours at reflux temperature in the presence of 20 mg of dichlorobistriphenylphosphine palladium(II), 5 mg of cuprous iodide (I) and 40 mg of triphenylphosphine, followed by cooling to the room temperature and removing the triethylamine. The product was extracted with toluene. The toluene layer was washed with 1N-hydrochloric acid and then with water, purified with silica gel colmun, and recrystalized thrice with ethanol to obtain 1.3 g of white crystalline Compound No.308 of this invention.

| | | | | |
|---|---|---|---|---|
| Elemental analysis,% | Observed | C: 78.89 | H: 8.12 | F: 3.81 |
| | Theoretical | C: 79.07 | H: 7.95 | F: 3.68 |

Example 39 [Compound No.414]

(1) In 150 ml of triethylamine, within nitrogen atmosphere, 10.0 g of 1-bromo-3-fluoro-4-iodobenzene and 4.6 g of 1-decyne were reacted for 8 hours at the room temperature in the presence of 18 mg of cuprous iodide(I) and 72 mg of dichlorobistriphenylphosphine palladium(II). After reaction, the triethyl amine was removed and the product was extracted with hexane. The hexane phase was washed with 1N-hydrochloric acid and then with water, followed by removing the hexane. The resulting liquid was dissolved in 200 ml of ethanol and hydrogenated using platinum dioxide. After confirming that no absorption of hydrogen was observed, the catalyst was removed, followed by removing the ethanol to obtain 8.9 g of 4-n-decyl-3-fluorobromobenzene.

(2) In 150 ml of triethylamine, within nitrogen atmosphere, 8.9 g of 4-n-decyl-3-fluoro-bromobenzene and 2.8 g of 3-methyl-1-butyne-3-ol were reacted for 8 hours at reflux temperature in the presence of 25 mg of cuprous iodide(I), 100 mg of dichlorobistriphenylphosphine palladium(II) and 200 mg of triphenylphosphine. After reaction and cooling to the room temperature, the triethyl amine was removed and the product was extracted with toluene. The toluene phase was washed with 1N-hydrochloric acid and then with water, and the toluene was removed. Then, the product was dissolved in methanol and treated with active carbon, followed by removing the methanol. To the resulting liquid, were added 350 ml dry toluene and 2.2 g of powdery sodium hydroxide, and heated for an hour to reflux temperature. After cooling, the toluene layer was washed with water, followed by removing the toluene to obtain 4.5 g of oily 4-n-decyl-3-fluorophenylacetylene.

(2) Example 38 was repeated except that 4-n-decyl-3-fluorophenylacetylene was used instead of 4-n-decyloxy-3-fluorophenylacetylene to obtain 1.1 g of Compound No.414 of the present invention.

| | | | | |
|---|---|---|---|---|
| Elemental | Observed | C: 81.45 | H: 8.33 | F: 3.91 |
| analysis,% | Theoretical | C: 81.60 | H: 8.20 | F: 3.80 |

Example 40 [Compound No.614]

(1) Into 200 ml of anhydrous THF, were dissolved 10.0 g of 2-fluoro-4-iodobenzoic acid prepared by oxidation of 2-fluoro-4-iodotoluene with potassium permanganate, 11.8 g of triphenylphosphine and 3.2 g of oa 2-methylbutanol, and cooled with ice bath to a temperature below 10°C. To this solution, were added 7.8 g of diethyl azodicarboxylate dropwise at a temperature below 10°C, and stirring was continued for an hour in that conditions and then for 24 hours at the room temperature, followed by removing the solvent. Hexane-soluble matter of the product was purified with silica gel column, followed by removing the hexane to obtain 11.1 g of oa 4-(2-methylbutyloxycarbonyl)-3-fluoroiodobenzene.

(2) In 40 ml of triethylamine, within nitrogen atmosphere, 1.5 g of oa 4-(2-methylbutyloxycarbonyl)-3-fluoroiodobenzene and 1.5 g of 2-n-decyloxy-6-ethynylnaphthalene were reacted for 8 hours at the room temperature in the presence of 12 mg of dichlorobistriphenylphosphine palladium(II) and 3 mg of cuprous iodide (I) as catalysts, followed by removing the triethylamine. The product was extracted with toluene. The toluene layer was washed with 1N-hydrochloric acid and then with water, purified with silica gel colmun, and recrystalized thrice with ethanol to obtain 1.3 g of white crystalline Compound No.614 of this invention.

| | | | | |
|---|---|---|---|---|
| Elemental | Observed | C: 79.14 | H: 8.20 | F: 3.51 |
| analysis,% | Theoretical | C: 79.07 | H: 7.95 | F: 3.68 |

Example 41 [Compound No.514]

Example 40 (2) was repeated except that 2-n-decyl-6-ethynylnaphthalene was used instead of 2-n-decyloxy-6-ethynylnaphthalene to obtain Compound No.514 of the present invention.

| | | | | |
|---|---|---|---|---|
| Elemental | Observed | C: 81.67 | H: 8.11 | F: 3.93 |
| analysis,% | Theoretical | C: 81.60 | H: 8.20 | F: 3.80 |

Example 42 [Compound No.602]

(1) Example 40 (1) was repeated except that 2-chloronicotinic acid was used instead of 2-fluoro-4-iodobenzoic acid to obtain oa 2-chloro-5-(2-methylbutyloxycarbonyl) pyridine.

(2) In 60 ml of triethylamine, within nitrogen atmosphere, 1.5 g of 2-chloro-5-(2-methylbutyloxycarbonyl)pyridine and 2.0 g of 2-n-decyloxy-6-ethynylnaphthalene were reacted for 10 hours at reflux temperature in the presence of 24 mg of dichlorobistriphenylphosphine palladium(II), 6 mg of cuprous iodide (I) and 48 mg of triphenylphosphine, followed by removing the triethylamine. The product was extracted with toluene. The toluene layer was washed with 1N-hydrochloric acid and then with water,

purified with silica gel colmun, and recrystalized thrice with ethanol to obtain 1.4 g of white crystalline Compound No.602 of this invention.

| Elemental analysis,% | | | | |
|---|---|---|---|---|
| | Observed | C: 79.43 | H: 8.16 | N: 2.72 |
| | Theoretical | C: 79.35 | H: 8.22 | N: 2.81 |

Example 43 [Compound No.502]

Example 42 (2) was repeated except that 2-n-decyl-6-ethynylnaphthalene was used instead of 2-n-decyloxy-6-ethynylnaphthalene to obtain Compound No.502 of the present invention.

| Elemental analysis,% | | | | |
|---|---|---|---|---|
| | Observed | C: 82.05 | H: 8.21 | F: 3.11 |
| | Theoretical | C: 81.98 | H: 8.49 | F: 2.90 |

Example 44 [Compound No.152]

In 60 ml of triethylamine, within nitrogen atmosphere, 1.5 g of 6-n-decyl-2-ethynylnaphthalene prepared in Example 14 (1) and 1.5 g of 4-(7-octenyloxy)-3-fluorobromobenzene prepared by reacting 8-bromo-1-octene with alkali metal 4-bromo-2-fluorophenolate were reacted for 8 hours at reflux temperature in the presence of 20 mg of dichlorobistriphenylphosphine palladium(II), 5 mg of cuprous iodide (I) and 40 mg of triphenylphosphine, followed by removing the triethylamine. The product was extracted with toluene. The toluene layer was washed with 1N-hydrochloric acid and then with water, purified with silica gel colmun, and recrystalized thrice with ethanol to obtain 1.2 g of white crystalline Compound No.152 of this invention.

| Elemental analysis,% | | | | |
|---|---|---|---|---|
| | Observed | C: 84.50 | H: 8.82 | F: 3.55 |
| | Theoretical | C: 84.37 | H: 8.79 | F: 3.71 |

Example 45 [Compound No.122]

In 30 ml of triethylamine, within nitrogen atmosphere, 1.5 g of 6-n-decyl-2-ethynylnaphthalene prepared in Example 14 (1) and 1.9 g of 4-(7-octenyloxy)-2,3-difluoroiodobenzene prepared by reacting 8-bromo-1-octene with alkali metal 2,3-difluoro-4-iodophenolate were reacted for 3 hours at the room temperature in the presence of 12 mg of dichlorobistriphenylphosphine palladium(II) and 3 mg of cuprous iodide (I) as the catalyst, followed by removing the triethylamine. The product was extracted with toluene. The toluene layer was washed with 1N-hydrochloric acid and then with water, purified with silica gel colmun, and recrystalized thrice with ethanol to obtain 1.3 g of Compound No.122 of this invention.

| Elemental analysis,% | | | | |
|---|---|---|---|---|
| | Observed | C: 81.33 | H: 8.47 | F: 7.33 |
| | Theoretical | C: 81.51 | H: 8.30 | F: 7.17 |

Example 46 [Compound No.165]

In 50 ml of triethylamine, within nitrogen atmosphere, 1.0 g of 6-n-nonyl-2-ethynylnaphthalene prepared in Example 15 (1) and 1.2 g of 4-n-nonyloxy-2-fluorobromobenzene prepared by reacting n-nonylbromide with alkali metal 4-bromo-3-fluorophenolate were reacted for 8 hours at reflux temperature in the presence of 12 mg of dichlorobistriphenylphosphine palladium(II), 4 mg of cuprous iodide (I) and 24 mg of triphenylphosphine,

followed by removing the triethylamine. The product was extracted with toluene. The toluene layer was washed with 1N-hydrochloric acid and then with water, purified with silica gel colmun, and recrystalized thrice with ethanol to obtain 0.8 g of Compound No.165 of this invention.

| | | | | |
|---|---|---|---|---|
| Elemental | Observed | C: 84.21 | H: 9.02 | F: 3.94 |
| analysis,% | Theoretical | C: 84.05 | H: 9.14 | F: 3.70 |

Example 47 [Compound No.44]

(1) In 150 ml of triethylamine, within nitrogen atmosphere, 5.6 g of 3-methyl-1-butyne-3-ol and 16.6 g of 2-bromo-5-n-decylpyridine prepared in Example 14 (1) were reacted for 6 hours at reflux temperature in the presence of 200 mg of dichlorobistriphenylphosphine palladium(II), 50 mg of cuprous iodide (I) and 400 mg of triphenylphosphine, followed by removing the triethylamine. The product was extracted with hexane. The hexane layer was washed with 1N-hydrochloric acid and then with water, and the hexane was removed. The product was dissolved in 300 ml dry toluene, followed by adding thereto 1.2 g of powdery sodium hydroxide and then heating for an hour to reflux temperature. After reaction and cooling to the room temperature, the toluene layer was washed with water, and then the toluene was removed. The resulting product was dissolved into methanol and treated with activated carbon, followed by removing the methanol to obtain 6.3 g of liquid 2-ethynyl-5-n-decylpyridine.

(2) In 60 ml of triethylamine, within nitrogen atmosphere, 1.5 g of 2-ethynyl-5-n-decylpyridine and 2.1 g of 6-bromo-2-(7-octenyloxy)naphthalane prepared by reacting 8-bromo-1-octene with alkali metal 6-bromo-2-naphtholate were reacted for 8 hours at reflux temperature in the presence of 20 mg of dichlorobistriphenylphosphine palladium(II), 5 mg of cuprous iodide (I) and 40 mg of triphenylphosphine, followed by removing the triethylamine. The product was extracted with toluene. The toluene layer was washed with 1N-hydrochloric acid and then with water, purified with silica gel colmun, and recrystalized thrice with ethanol to obtain 1.1 g of Compound No.44 of this invention.

| | | | | |
|---|---|---|---|---|
| Elemental | Observed | C: 84.62 | H: 9.23 | N: 2.99 |
| analysis,% | Theoretical | C: 84.85 | H: 9.09 | N: 2.83 |

Example 48 [Compound No.52]

(1) Example 8 (4) and (5) were repeated except that 1-octyne was used instead of 1-nonyne in Example 8 (4) to obtain 2,3-difluoro-4-octylphenyl trifluoromethanesulfonate.

(2) In 80 ml of triethylamine, within nitrogen atmosphere, 1.5 g of 3-methyl-1-butyne-3-ol and 5.6 g of 2,3-difluoro-4-octylphenyl trifluoromethanesulfonate were reacted for 8 hours at reflux temperature in the presence of 52 mg of dichlorobistriphenylphosphine palladium(II), 13 mg of cuprous iodide (I) and 104 mg of triphenylphosphine, followed by removing the triethylamine. The product was extracted with hexane. The hexane layer was washed with 1N-hydrochloric acid and then with water, and the hexane was removed. The product was dissolved in 300 ml dry toluene, followed by adding thereto 1.2 g of powdery sodium hydroxide and then heating for an hour to reflux temperature. After reaction and cooling to the room temperature, the toluene layer was washed with water, and then the toluene was removed. The resulting product was dissolved into methanol and treated with active carbon, followed by removing the methanol to obtain 2.0 g of liquid 4-n-octyl-2,3-difluorophenylacetylene.

(3) In 50 ml of triethylamine, within nitrogen atmosphere, 1.0 g of 4-n-octyl-2,3-difluorophenylacetylene and 1.5 g of 6-bromo-2-(9-decenyloxy)naphthalane were reacted for 8 hours at reflux temperature in the presence of 16 mg of dichlorobistriphenylphosphine palladium(II), 4 mg of cuprous iodide (I) and 32 mg of triphenylphosphine, followed by removing the triethylamine. The product was extracted with toluene. The toluene layer was washed with 1N-hydrochloric acid and then with water, purified with silica gel colmun, and recrystalized thrice with ethanol to obtain 1.0 g of white crystalline Compound No.52 of this invention.

| | | | | |
|---|---|---|---|---|
| Elemental | Observed | C: 81.29 | H: 8.32 | F: 7.41 |
| analysis,% | Theoretical | C: 81.51 | H: 8.30 | F: 7.17 |

Example 49 [Compound No.69]

(1) In 150 ml of triethylamine, within nitrogen atmosphere, 10.0 g of 1-bromo-3-fluoro-4-iodobenzene and 4.6 g of 1-decyne were reacted for 8 hours at the room temperature in the presence of 72 mg of dichlorobistriphenylphosphine palladium(II) and 18 mg of cuprous iodide (I), followed by removing the triethylamine. The product was extracted with hexane. The hexane layer was washed with 1N-hydrochloric acid and then with water, and the hexane was removed. The product was dissolved in 200 ml of ethanol, and hydrogenated using 0.3 g of platinum dioxide. After confirming that no absorption of hydrogen was observed, the catalyst was removed by filtration, followed by removing the ethanol to obtain 8.8 g of 4-n-decyl-3-fluorobromobenzene.
(2) Example 1 (6) was repeated except that 4-n-decyl-3-fluoro-bromobenzene was used instead of 2-bromo-5-n-nonylpyridine to obtain Compound No.69 of this invention.

| | | | | |
|---|---|---|---|---|
| Elemental | Observed | C: 84.21 | H: 9.02 | F: 3.93 |
| analysis,% | Theoretical | C: 84.05 | H: 9.14 | F: 3.70 |

Example 50 [Compound No.254]

(1) In 150 ml of triethylamine, within nitrogen atmosphere, 3.3 g of 3-methyl-1-butyne-3-ol and 10.0 g of 4-n-octyloxy-3-fluorobromobenzene prepared by reacting n-octylbromide with alkali metal 4-bromo-2-fluorophenol were reacted for 8 hours at reflux temperature in the presence of 28 mg of cuprous iodide(I), 112 mg of dichlorobistriphenylphosphine palladium(II) and 224 mg of triphenylphosphine. After reaction and cooling to the room temperature, the triethyl amine was removed and the product was extracted with hexane. The hexane phase was washed with 1N-hydrochloric acid and then.with water, followed by removing the hexane. Then, the resulting liquid was dissolved in 350 ml dry toluene, and 2.6 g of powdery sodium hydroxide were added thereto, followed by heating for an hour to reflux temperature. After cooling, the toluene layer was washed with water, followed by removing the toluene to obtain 5.2 g of oily 4-n-octyloxy-3-fluorophenylacetylene.
(2) In 60 ml of triethylamine, within nitrogen atmosphere, 1.5 g of 4-n-octyloxy-3-fluorophenylacetylene and 2.0 g of 6-bromo-2-n-octyloxynaphthalene prepared by reacting n-octylbromide with alkali metal 6-bromo-2-naphtholate were reacted for 8 hours at reflux temperature in the presence of 5 mg of cuprous iodide(I), 20 mg of dichlorobistriphenylphosphine palladium(II) and 40 mg of triphenylphosphine. After cooling to the room temperature, the triethyl amine was removed and the product was extracted with toluene. The toluene phase was washed with 1N-hydrochloric acid and then with water, purified with silica gel column and then recrystalized thrice with ethanol to obtain 1.3 g of white crystalline Compound No.254 of the present invention.

| | | | | |
|---|---|---|---|---|
| Elemental | Observed | C: 81.35 | H: 8.41 | F: 4.03 |
| analysis,% | Theoretical | C: 81.28 | H: 8.57 | F: 3.78 |

Example 51 [Compound No.305]

Example 33 was repeated except that trifluoromethanesulfonate of octyl 6-hydroxy-2-naphthoate was used instead of trifluoromethanesulfonate of 2-methylbutyl 6-hydroxy-2-naphthoate to obtain Compound No.305 of the present invention.

| | | | | |
|---|---|---|---|---|
| Elemental analysis,% | Observed | C: 76.83 | H: 8.14 | F: 6.69 |
| | Theoretical | C: 77.08 | H: 7.99 | F: 6.60 |

Example 52 [Compound No.317]

Example 31 was repeated except that trifluoromethanesulfonate of octyl 6-hydroxy-2-naphthoate was used instead of trifluoromethanesulfonate of 2-methylbutyl 6-hydroxy-2-naphthoate to obtain Compound No.317 of the present invention.

| | | | | |
|---|---|---|---|---|
| Elemental analysis,% | Observed | C: 80.01 | H: 8.53 | N: 2.66 |
| | Theoretical | C: 79.85 | H: 8.69 | N: 2.59 |

Example 53 [Compound No.317]

Example 35 (2) was repeated except that trifluoromethanesulfonate of octyl 6-hydroxy-2-naphthoate was used instead of trifluoromethanesulfonate of 6-hydroxy-2-(2-methylbutyl)naphthoate to obtain Compound No.317 of the present invention.

Example 54 [Compound No.511]

(1) Example 25 (2) was repeated except that 2-n-decyl-6-ethynylnaphthalene as prepared in Example 26 (4) was used instead of 2-n-decyloxy-6-ethynylnaphthalene to prepare a compound of the formula : DEC-NAP-#-FPhF-H.

(2) Into 100 ml of anhydrous THF, were dissolved 4.5 g of this compound and 5.0 g of N,N,N',N'-tetramethylmethylenediamine, and cooled with dry ice-acetone bath to a temperature below -60°C. Then, 24 ml of 15% solution of n-butyl lithium in hexane were added dropwise thereto at a temperature below -60°C and stirring was continued for 3 hours in that conditions, followed by browing thereinto carbon dioxide at a temperature below -60°C. Thereafter, the dry ice-acetone bath was removed, and then stirring was continued until the temperature reached 0°C. To the reaction product, were added water and then 1N-hydrochloric acid, followed by extracting the resulting product with ether. The ether layer was washed with water, followed by removing the ether to obtain 4.4 g of a compound of the formula : DEC-NAP-#-FPhF-COOH.

(2) Into 50 ml of anhydrous THF, were dissolved 2.0 g of this compound, 1.4 g of triphenylphosphine and 0.7 g of n-octanol, and cooled with iced water bath to a temperature below 10°C. To this solution, was deed dropwise 1.0 g of diethyl azodicarboxylate at a temperature below 10°C and stirring was continued for 1 hour in that conditions and then for 24 hours at the room temperature, followed by removing the solvent. Hexane-soluble matter was dissolved into toluene, purified with silica gel colmun, and then recrystalized thrice with ethanol to obtain 1.3 g of white crystalline Compound No.511 of this invention.

| | | | | |
|---|---|---|---|---|
| Elemental analysis,% | Observed | C: 79.44 | H: 8.13 | F: 7.01 |
| | Theoretical | C: 79.29 | H: 8.21 | F: 6.79 |

Example 55 [Compound No.714]

(1) Example 26 (1)-(3) were repeated except that 1-octyne was used instead of 1-decyne in Example 26 (1) to prepare trifluoromethanesulfonate of 6-n-octyl-2-hydroxynaphthalene.

(2) In 60 ml of triethylamine, within nitrogen atmosphere, 2.1 g of trifluoromethanesulfonate of 6-n-octyl-2-hydroxy-naphthalene and 1.5 g of 2,3-difluoro-4-n-decylphenylacetylene as prepared in Example 35 (2) were reacted for 8 hours at reflux temperature in the presence of 16 mg of dichlorobistriphenylphosphine palladium(II), 4 mg of cuprous iodide (I) and 32 mg of triphenylphosphine, followed by cooling to the room

temperature and removing the triethylamine. The product was extracted with toluene. The toluene layer was washed with 1N-hydrochloric acid and then with water, purified with silica gel column, and recrystalized thrice with ethanol to obtain 1.2 g of white crystalline Compound No.714 of this invention.

| | | | | |
|---|---|---|---|---|
| Elemental analysis,% | Observed | C: 83.61 | H: 9.05 | F: 7.34 |
| | Theoretical | C: 83.72 | H: 8.92 | F: 7.36 |

Example 56

According to the following formulation, liquid crystal compositions were prepared, having response speed of 45 micro second and being free from zigzag deffects.

| | % by weight |
|---|---|
| Compound No.315 | 10.0 |
| DEC-Pyr>-NAP-O-$CH_2$*CH(Me)Et | 24.4 |
| NON-Pyr>-NAP-O-OCT | 9.0 |
| DOD-O-Ph-#-Ph-COO-$CH_2$*CH(Me)Et | 6.65 |
| DEC-O-Ph-#-PhF-COO-$(CH_2)_3$*CH(Me)Et | 6.65 |
| DEC-O-Ph-#-PhF-COO-$(CH_2)_5$*CH(Me)Et | 6.65 |
| DEC-O-Ph-#-FPhF-COO-$(CH_2)_5$*CH(Me)Et | 6.65 |
| DEC-O-Ph-#-PhF-O-HEX | 10.0 |
| OCT-<Pyr-#-FPhF-O-DEC | 10.0 |
| DEC-<Pyr-#-FPhF-O-OCT | 10.0 |

Phase transition temperatures of the compounds and composition of Examples 1-55 were as shown in Table 9, wherein the following abbreviated words are used.

Phase transition temperatures of compounds and compositions of Examples 1-49 were as shown in Table 13, wherein the following abbreviated words are used.

Cry: crystal phase; $S_C$ : smectic C phase;

$S_C^*$ : chiral smectic C phase; $S_A$ : smectic A phase;

Nem: nematic phase; Ch :cholesteric phase;

Iso: isotropic liquid phase; ( ) :monotropic phase;

• : the phase is present; – : the phase is not present

Table 9

| Example No. | Compound No. | Phase transition temperatures (°C) | | | | |
|---|---|---|---|---|---|---|
| | | Cry | $S_C$ ($S_C^*$) | $S_A$ | Nem (Ch) | Iso |
| 1 | 10 | · 74.8 | · 77.8 | – | · 112.8 | · |
| 2 | 9 | · 77.7 | (· 74.2) | – | · 108.7 | · |
| 3 | 8 | · 85.8 | (· 62.9) | – | · 115.4 | · |
| 4 | 4 | · 78.5 | (· 55.5) | – | · 109.1 | · |
| 5 | 5 | · 70.8 | (· 67.7) | – | · 110.9 | · |
| 6 | 12 | · 81.8 | (· 61.4) | – | · 112.0 | · |
| 7 | 13 | · 77.7 | (· 74.2) | – | · 108.7 | · |
| 8 | 26 | · 54.0 | · 67.3 | – | · 98.7 | · |
| 9 | 144 | · 58.6 | · 59.4 | – | · 94.1 | · |
| 10 | 133 | · 58.5 | · 65.3 | – | · 92.9 | · |
| 11 | 136 | · 61.3 | (· 58.6) | – | · 97.8 | · |
| 12 | 141 | · 67.3 | (· 65.1) | – | · 95.3 | · |
| 13 | 114 | · 51.0 | · 59.4 | – | · 100.9 | · |
| 14 | 103 | · 56.5 | (· 47.0) | – | · 100.1 | · |
| 15 | 106 | · 56.8 | (· 46.0) | – | · 105.1 | · |
| 16 | 111 | · 58.5 | · 58.6 | – | · 102.1 | · |
| 17 | 402 | · 74.0 | (· 51.4) | · 81.2 | · 82.8 | · |
| 18 | 211 | · 85.0 | · 89.4 | – | · 120.7 | · |
| 19 | 237 | · 51.8 | · 79.4 | – | · 117.0 | · |
| 20 | 222 | · 61.4 | · 79.7 | – | · 119.1 | · |
| 21 | 62 | · 69.1 | – | – | ·Ch72.6 | · |
| 22 | 256 | · 69.1 | – | – | – | · |
| 23 | 247 | · 90.3 | – | – | (·Ch90.2) | · |

Table 9 (continued)

| Example No. | Compound No. | Phase transition temperatures (°C) | | | | |
|---|---|---|---|---|---|---|
| | | Cry | $S_C$ ($S_C^*$) | $S_A$ | Nem (Ch) | Iso |
| 24 | 63 | · 41.4 | – | – | – | · |
| 25 | 252 | · 45.5 | – | · 52.6 | – | · |
| 26 | 168 | · 20.0 | – | – | – | · |
| 27 | 255 | · 77.0 | – | – | ·Ch85.3 | · |
| 28 | 163 | · 29.8 | – | – | – | · |
| 29 | 401 | · 52.5 | · 63.2 | · 77.5 | – | · |
| 30 | 403 | · <RT | – | · 46.4 | – | · |
| 31 | 314 | · 66.0 | (· 59.4) | – | – | · |
| 32 | 315 | · 73.6 | – | – | – | · |
| 33 | 302 | · 77.7 | – | (· 75.0) | ·Ch92.7 | · |
| 34 | 303 | · 56.7 | – | – | – | · |
| 35 | 409 | · -2.4 | – | – | – | · |
| 36 | 609 | · 49.5 | – | · 58.6 | – | · |
| 37 | 509 | · 34.5 | – | – | – | · |
| 38 | 308 | · 53.8 | – | · 70.1 | ·Ch82.2 | · |
| 39 | 414 | · 55.0 | – | – | (·Ch37.4) | · |
| 40 | 614 | · 68.5 | – | · 96.5 | – | · |
| 41 | 514 | · 51.5 | – | · 63.0 | · 64.3 | · |
| 42 | 602 | · 96.3 | – | (· 87.1) | (·Ch92.4) | · |
| 43 | 502 | · 97.0 | – | – | – | · |
| 44 | 152 | · 46.9 | · 49.2 | · 66.3 | · 88.3 | · |
| 45 | 122 | · 46.4 | · 47.9 | – | · 94.4 | · |
| 46 | 165 | · 54.5 | – | – | · 100.4 | · |

Table 9 (continued)

| Example No. | Compound No. | Phase transition temperatures (°C) Cry | | $S_C$ ($S_C^*$) | | $S_A$ | | Nem (Ch) | | Iso |
|---|---|---|---|---|---|---|---|---|---|---|
| 47 | 44 | · | 68.7 | · | 72.9 | – | | · | 102.5 | · |
| 48 | 52 | · | 54.4 | · | 57.5 | · | 75.8 | · | 92.4 | · |
| 49 | 69 | · | 73.2 | – | | · | 90.0 | · | 94.5 | · |
| 50 | 254 | · | 89.8 | – | | – | | · | 122.7 | · |
| 51 | 305 | · | 57.8 | · | 76.1 | · | 86.8 | · | 91.9 | · |
| 52 | 317 | · | 79.7 | – | | (· | 52.4 | · | 68.6) | · |
| 53 | 411 | · | 43.7 | – | | – | | – | | · |
| 54 | 511 | · | 66.7 | – | | (· | 64.3 | · | 65.0) | · |
| 55 | 714 | · | 49.5 | – | | – | | · | 70.3 | · |
| – | 420 | · | 56.4 | – | | · | 58.5 | – | | · |
| 56 | | · | <RT | · | 33 | · | 69 | – | | · |

(Note) <RT : below room temperature

IR ($cm^{-1}$) and NMR (ppm) spectra of some of the compounds of Examples 18-55 are as follows:

Example 18, Compound 211

IR 2922 2854 2300 1636 1603 1473 1392 1303 1261 1195 1085 1017 859 804
NMR 0.86-0.95(m,6H) 1.20-1.67(m,24H) 1.76-1.89(m,4H) 4.00-4.09(m,4H) 6.65-6.73(m,1H) 7.06-7.24(m,3H) 7.53 (dd,1H) 7.68(t,2H) 7.98(s,1H)
$^{19}$F-NMR -58.1(dd,1F) -82.9(dd,1F)

Example 19, Compound 237

IR 2922 2856 1603 1473 1394 1303 1263 1195 1089 1019 893 861 806
NMR 0.89(t,3H) 1.18-1.62(m,20H) 1.77-1.94(m,4H) 1.99-2.15(m,2H) 4.00-4.14(m,2H) 4.92-5.06(m,2H) 5.73-5.89 (m,1H) 6.64-6.76(m,1H) 7.06-7.26(m,3H) 7.54(dd,1H) 7.71 (t,2H) 7.99(s,1H)
$^{19}$F-NMR -58.1(dd,1F) -82.9(dd,1F)

Example 20, Compound 222

IR 2926 2310 1624 1603 1514 1464 1388 1299 1267 1224 1195 1083 855 806
NMR 0.88(t,3H) 1.20-1.58(m,20H) 1.77-1.89(m,4H) 1.98-2.09(m,2H) 4.00-4.10(m,4H) 4.90-5.04(m,2H) 5.75-5.89 (m,1H) 6.66-6.74(m,1H) 7.08-7.26(m-,3H) 7.52(dd,1H) 7.68 (t,2H) 7.98(d,1H)
$^{19}$F-NMR -58.1(dd,1F) -82.9(dd,1F)

Example 21, Compound 62

IR 2924 2854 2214 1601 1499 1464 1394 1261 1212 1174 1031 855 818
NMR 0.88(t,3H) 0.98(t,3H) 1.06(d,3H) 1.20-1.41(m,16H) 1.54-1.70(m,2H) 1.87-2.02(m,1H) 2.62(t,2H) 3.81-3.97 (m,2H) 7.11(d,1H) 7.18 (dd,1H) 7.43-7.53(m,2H) 7.59 (dd,1H) 7.70(t,2H) 8.05(s,1H) 8.47(s,1H)

Example 22, Compound 256

IR 2922 2854 2320 1597 1504 1466 1373 1257 1209 1168 1112 1033 891 855 814
NMR 0.89(t,3H) 0.99(t,3H) 1.07(d,3H) 1.19-1.52(m,14H) 1.55-1.72(m,2H) 1.73-1.86(m,2H) 1.86-2.03(m,1H) 3.82-3.99(m,2H) 4.31(t,2H) 6.73(d,1H) 7.08-7.23(m,2H) 7.52 (dd,1H) 7.66-7.77(m,3H) 7.96(s,1H) 8.38(d,1H)

Example 23, Compound 247

IR 2926 2200 1603 1512 1464 1388 1296 1265 1224 1195 1083 994 893 857 801
NMR 0.89(t,3H) 0.98(t,3H) 1.07(d,3H) 1.20-1.71(m,16H) 1.78-2.01(m,3H) 3.81-3.99(m,2H) 4.05(t,2H) 6.66-6.75 (m,1H) 7.11(d,1H) 7.15-7.27(m,2H) 7.54(dd,1H) 7.70(t,2H) 7.79(s,1H)
$^{19}$F-NMR -57.9(dd,1F) -82.7(dd,1F)

Example 24, Compound 63

IR 2922 2856 2216 1601 1468 1381 1261 1212 1178 1069 953 893 857 822
NMR 0.82-0.92(m,6H) 1.20-1.42(m,25H) 1.53-1.83(m,4H) 2.21(t,2H) 4.44 4.56(m,1H) 7.06-7.16(m,2H) 7.42-7.50 (m,2H) 7.57(dd,1H) 7.63-7,73(m,2H) 8.02(s,1H) 8.45(s,1H)

Example 25, Compound 252

IR 2928 2858 2360 1630 1603 1510 1468 1386 1296 1195 1067 893 857 799
NMR 0.83-0.94(m,6H) 1.20-1.55(m,25H) 1.70-1.90(m,4H) 4.07(t,2H) 4.35-4.95(m,1H) 6.66-6.75(m,1H) 7.08-7.23 (m,3H) 7.53(dd,1H) 7,68(t,2H) 7.98(s,1H)
$^{19}$F-NMR -57,8(dd,1F) -81.7(dd,1F)

Example 26, Compound 168

IR 2930 2858 2210 1632 1514 1466 1379 1296 1120 1056 891 812 723
NMR 0.83-0.95(m,6H) 1.20-1.55(m,25H) 1.58-1.75(m,4H) 2,75(t,2H) 4.37-4.48(m,1H) 6.79-6.88(m,1H) 7.14-7.22 (m,1H) 7.35(dd,1H) 7.57-7.63(m,1H) 7.80(t,2H) 7.94(s,1H)
$^{19}$F-NMR -65.7(dd,1F) -81.4(dd,1F)

Example 27, Compound 255

IR 2924 2856 2210 1626 1601 1518 1468 1392 1319 1267 1238 1189 1139 1031 957 857 820
NMR 0.89(t,3H) 0.99(t,3H) 1.07(d,3H) 1.21-1.42(m,13H) 1.42-1.53(m,2H) 1.57-1.70(m,1H) 1.79-1.90(m,2H) 1.90-2.11(m,1H) 3.86-3.99(m,2H) 4.05(t,2H) 6.92(t,1H) 7.12 (d,1H) 7.19(dd,1H) 7.24-7.34(m,2H) 7.52(dd,1H) 7.69 (t,2H) 7.96(s,1H)
$^{19}$F-NMR -58.4(t,1F)

Example 28, Compound 153

IR 2928 2858 2214 1601 1514 1466 1379 1296 1270 1234 1131 975 888 814
NMR 0.83-0.95(m,6H) 1.20-1.48(m,25H) 1.60-1.87(m 4H) 2.75(t,2H) 4,32-4.45(m,1H) 6.93(t,1H) 7.24-7.39(m,3H) 7.49-7.61(m,2H) 7.74(d,2H) 7.99(s,1H)
$^{19}$F-N-MR -57.3(t,1F)

Example 29, Compound 401

IR 2926 2856 2200 1715 1555 1464 1365 1286 1228 1181 1093 919 895 818 752

NMR 0.82-0.93(m,9H) 1.12-1,50(m,19H) 1.54-1.68(m,2H) 1.72-1.89(m,2H) 2.62(t,2H) 4.36(t,2H) 7.45-7.53(m,2H) 7.67(dd,1H) 7.84(d,2H) 7.91(d,1H) 8.08(dd,1H) 8.12(s,1H) 8.46(s,1H) 8.56(s,1H)

Example 30, Compound 403

IR 2924 2856 2310 1709 1468 1586 1286 1226 1183 1093 903 822 754
NMR 0.83-0.92(m,6H) 1.19-1.42(m,25H) 1.55-1.72(m,3H) 1.72-1.87(m,1H) 2.62(t,2H) 5.15-5.25(m,1H) 7.45-7.52 (m,2H) 7.67(dd,1H) 7.84(d,1H) 7.92(d,1H) 8.07(dd,1H) 8.13(s,1H) 8.47(s,1H) 8.56(s,1H)

Example 31, Compound 314

IR 2920 2216 1719 1607 1499 1359 1276 1220 1183 1137 1098 895 828 748
NMR 0.88(t,3H) 0.98(t,3H) 1.03(d,3H) 1.17-1.61(m,16H) 1.71-1.82(m,2H) 1.82-1.98(m,1H) 4.15-4.35(m,4H) 6.73 (d,1H) 7.61(dd,1H) 7.71(dd,1H) 7.81-7.95(m,2H) 8.04-8.10 (m,2H) 8.38(d,1H) 8.57(s,1H)

Example 32, Compound 315

IR 2926 2856 2200 1717 1595 1497 1363 1270 1224 1185 1098 1064 891 833 752
NMR 0.81-0.94(m,6H) 1.18-1.50(m,18H) 1.58-1.71(m,1H) 1.71-1.85(m,3H) 4.30(t,2H) 5.16-5.77(m,2H) 6.73(dd,1H) 7.60(dd,1H) 7.71(dd,1H) 7.82-7.95(m,2H) 8.03-8.10(m,2H) 8.37(d,1H) 8.55(s,1H)

Example 33, Compound 302

IR 2928 2216 1715 1626 1470 1394 1303 1232 1181 1087 984 888 814 723
NMR 0.89(t,3H) 1.01(t,3H) 1.08(d,3H) 1.19-1.68(m,16H) 1.77-2.00(m,3H) 4.07(t,2H) 4.16-4.35(m,2H) 6.67-6.80 (m,1H) 7.19-7.31(m,1H) 7.65(dd,1H) 7.82-7.98(m,2H) 8.05-8.15(m,2H) 8.59(s,1H)
$^{19}$F-NMR -57.6(dd,1F) -82.5(dd,1F)

Example 34, Compound 303

IR 2924 2856 2216 1709 1628 1516 1470 1383 1301 1234 1183 1081 911 801 750
NMR 0.83-0.96(m,6H) 1.20-1.72(m,26H) 1.73-1.90(m,3H) 4.08(t,2H) 5.17-5.29(m,1H) 6.68-6.77(m,1H) 7.18-7.28 (m,1H) 7.64(dd,1H) 7.83-7.95(m,2H) 8.06-8.12(m,2H) 8.57(d,1H)
$^{19}$F-NMR -57.8(dd,1F) -82.7(dd,1F)

Example 35, Compound 409

IR 2922 2854 2310 1713 1630 1468 1386 1274 1230 1197 1098 959 891 816 752
NMR 0.84-0.93(m,6H) 1.20-1.48(m,25H) 1.56-1.72(m,3H) 1.72-1.88(m,1H) 2.67(t,2H) 5.18-5.29(m,1H) 6.89-6.98 (m,1H) 7.19-7.28(m,1H) 7.64(dd,1H) 7.83-7.96(m,2H) 8.07-8.12(m,2H) 8.58(s,1H)
$^{19}$F-NMR -59.7(dd,1F) -67.4(dd,1F)

Example 36, Compound 609

IR 2924 2856 2214 1717 1622 1462 1392 1299 1232 1178 1116 1069 893 853 777
NMR 0.86-0.96(m,6H) 1.22-1.78(m,27H) 1-78-1.95(m,2H) 4.08(t,2H) 5.13-5.25(m,1H) 7.12(d,1H) 7.19(dd,1H) 7.29-7.38(m,1H) 7.55(dd,1H) 7.63-7.78(m,3H) 8.05(s,1H)
$^{19}$F-NMR -58.2(dd,1F) -59.0(dd,1F)

Example 37, Compound 509

IR 2928 2856 2216 1715 1626 1458 1381 1303 1222 1135 1021 886 816 777
NMR 0.83-0.95(m,6H) 1.20-1.49(m,25H) 1.61-1.81(m,4H) 2.78(t,2H) 5.13-5.25(m,1H) 7.31-7.41(m,2H) 7.55-7.64 (m,2H) 7.64-7.72(m,1H) 7.78(dd,1H) 8.08(s,1H)
$^{19}$F-NMR -58.0(dd,1F) -59.0(dd,1F)

Example 38, Compound 308

IR 2926 2854 2208 1717 1615 1518 1473 1270 1222 1197 1133 1096 1017 886 814 784

NMR 0.89(t,3H) 0.99(t,3H) 1.04(d,3H) 1.18-1.66(m,12H) 1.76-2.00(m,3H) 4.04(t,2H) 4.18-4.32(m,2H) 6.93(t,1H) 7.25-7.35 (m,2H) 7.61(dd,1H) 7.81-7.96(m,2H) 8.03-8.13 (m,2H) 8.57(s,1H)

$^{19}$F-NMR -58.2(t,1F)

Example 39, Compound 414

IR 2922 2362 1717 1628 1557 1510 1470 1410 1278 1203 1100 971 893 822 746

NMR 0.88(t,3H) 0.98(t,3H) 1.05(d,3H) 1.18-1.39(m,14H) 1.51-1.67(m,4H) 1.84-1.97(m,1H) 2.63(t,2H) 4.13-4.31 (m,2H) 7.13-7.31(m,3H) 7.61(dd,1H) 7.81-7.95(m,2H) 8.03-8.10(m,2H) 8.57(s,1H)

$^{19}$F-NMR -42.6(t,1F)

Example 40, Compound 614

IR 2926 2204 1711 1613 1470 1412 1278 1222 1191 1133 1017 975 843 775

NMR 0.88(t,3H) 0.95(t,3H) 1.02(d,3H) 1.19-1.58(m,16H) 1.78-1.90(m,3H) 4.06(t,2H) 4.10-4.28(m,2H) 7.10(d,1H) 7.17(dd,1H) 7.25-7.39(m,2H) 7.51(dd,1H) 7.65-7.75(m,2H) 7.92(t,1H) 7.99(s,1H)

$^{19}$F-NMR -33.6(t,1F)

Example 41, Compound 514

IR 2924 2856 2210 1715 1615 1555 1466 1415 1288 1139 977 895 824 775

NMR 0.86(t,3H) 0.95(t,3H) 1.01(d,3H) 1.18-1.42(m,14H) 1.42-1.62(m,2H) 1.62-1.78(m,2H) 1.78-1.94(m,2H) 2.78 (t,2H) 4.10-4.28(m,2H) 7.27-7.42(m,3H) 7.53(dd,1H) 7.60 (s,1H) 7.71-7.79(m,2H) 7.94(t,1H) 8.04(s,1H)

$^{19}$F-NMR -33.4(t,1F)

Example 42, Compound 602

IR 2922 2854 2216 1715 1589 1468 1392 1296 1172 1125 1019 901 853 820 779

NMR 0.86(t,3H) 0.90-1.15(m,6H) 1.15-1.76(m,16H) 1.76-1.98(m,3H) 4.08(t,2H) 4.11-4.32(m,2H) 7.06-7.23(m,2H) 7.55-7.81 (m,4H) 8.07(s,1H) 8.28(dd,1H) 9.22(s,1H)

Example 43, Compound 502

IR 2926 2856 2212 1717 1589 1468 1381 1294 1125 1015 978 895 824 781

NMR 0.88(t,3H) 0.91-1.05(m,6H) 1.15-1.60(m,15H) 1.62-1.78(m,3H) 1.82-1.96(m,2H) 2.76(t,2H) 4.12-4.30(m,2H) 7.37(dd,1H) 7.56-7.66(m,3H) 7.72-7.80(dd,2H) 8.12(s,1H) 8.29(dd,1H) 9.23(d,1H)

Example 44, Compound 152

IR 2920 2852 2300 1615 1522 1473 1427 1303 1276 1238 1131 1002 891 822

NMR 0.88(t,3H) 1.18-1.55(m,20H) 1.63-1.76(m,2H) 1.76-1.88(m,2H) 2.02-2.10(m,2H) 2.76(t,2H) 4.04(t,2H) 4.92-5.05(m,2H) 5.76-5.89(m,1H) 6.88-6.95(m,1H) 7.25-7.37 (m,3H) 7.51(dd,1H) 7.58(s,1H) 7.73(d,2H) 7.98(s,1H)

$^{19}$F-NMR -58.6(t,1F)

Example 45, Compound 122

IR 2920 2852 2231 1636 1601 1516 1468 1299 1089 998 886 818

NMR 0.88(t,3H) 1.19-1.54(m,20H) 1.62-1.77(m,2H) 1.77-1.89(m,2H) 2.02-2.13(m,2H) 2.76(t,2H) 4.04(t,2H) 4.91-5.05(m,2H) 5.74-5.89(m,1H) 6.66-6.74(m,1H) 7.17-7.27 (m,1H) 7.35(dd,1H) 7.52-7.61(m,2H) 7.74(d,2H) 8.03(s,1H)

$^{19}$F-NMR -58.0(dd,1F) -82.8(dd,1F)

Example 46, Compound 165

IR 2924 2854 2216 1628 1512 1466 1429 1342 1234 1172 1096 957 891 818
NMR 0.83-0.93(m,6H) 1.19-1.50(m,24H) 1.62-1.73(m,4H) 2.75(t,2H) 3.95(t,2H) 6.62-6.72(m,2H) 7.35(dd,1H) 7.40-7.49(m,1H) 7.52-7.62(m,2H) 7.73(d,2H) 8.02(s,1H)
$^{19}$F-NMR -32.0(t,1F)

Example 47, Compound 44

IR 2924 2856 2214 1624 1603 1464 1388 1259 1209 1172 1023 909 861 812
NMR 0.88(t,3H) 1.19-1.53(m,20H) 1.56-1.68(m,2H) 1.77-1.90(m,2H) 2.01-2.12(m,2H) 2.61(t,2H) 4.07(t,2H) 4.88-5.06(m,2H) 5.74-5.89(m,1H) 7.08(d,1H) 7.15(dd,1H) 7.42-7.51(m,2H) 7.58(dd,1H) 7.68(t,2H) 8.02 (s,1H) 8.45(s,1H)

Example 48, Compound 52

IR 2922 2854 2216 1624 1603 1466 1394 1265 1222 1183 1120 1027 893 851 812
NMR 0.89(t,3H) 1.22-1.57(m,20H) 1.57-1.69(m,2H) 1.79-1.92(m,2H) 2.01-2.11(m,2H) 2.18(t,2H) 4.08(t,2H) 4.92-5.07(m,2H) 5.75-5.90(m,1H) 6.89-6.95(m,1H) 7.10-7.27 (m,3H) 7.55(dd,1H) 7.72(t,2H) 8.01(s,1H)
$^{19}$F-NMR -60.1(dd,1F) -67.6(dd,1F)

Example 49, Compound 69

IR 2918 2852 2210 1632 1562 1470 1398 1270 1226 1170 1123 1025 975 847 723
NMR 0.82-0.96(m,6H) 1.21-1.42(m,22H) 1.42-1.66(m,4H) 1.79-1.92(m,2H) 2.63(t,2H) 4.07(t,2H) 7.07-7.30(m,5H) 7.52(dd,1H) 7-63-7.74(m,2H) 7.97(s,1H)
$^{19}$F-NMR -42.9(t,1F)

Example 50, Compound 254

IR 2924 2856 2216 1625 1601 1518 1463 1390 1318 1362 1234 1183 1135 1029 957 856 822
NMR 0.82-0.98(m,6H) 1.18-1.42(m,16H) 1.42-1.56(m,4H) 1.76-1.92(m,4H) 4.00-4.11(m,4H) 6.92(t,1H) 7.10(d,1H) 7.18(dd,1H) 7.23-7.32(m,2H) 7.52(dd,1H) 7.69(t,2H) 7.95 (s,1H)
$^{19}$F-NMR -58.4(t,1F)

Example 51, Compound 305

IR 2920 2856 2210 1709 1630 1518 1475 1408 1307 1280 1207 1089 888 808 716
NMR 0.82-0.99(m,6H) 1.18-1.55(m,24H) 1.74-1.92(m,4H) 4.06(t,2H) 4.37(t,2H) 6.66-6.80(m,1H) 7.18-7.29(m,1H) 7.63(dd,1H) 7.81-7.99(m,2H) 8.09(d,2H) 8.58(s,1H)
$^{19}$F-NMR -57.8(dd,1F) -82.7(dd,1F)

Example 52, Compound 317

IR 2924 2856 2208 1711 1605 1495 1475 1367 1286 1181 1098 1000 890 824 752
NMR 0.82-0.94(m,6H) 1.18-1.52(m,24H) 1.72-1.88(m,4H) 4.31(t,2H) 4.37(t,2H) 6.73(d,1H) 7.61(dd,1H) 7.68-7.73 (m,1H) 7.81-7.93(m,2H) 8.02-8.12(m,2H) 8.38(d,1H) 8.56 (s,1H)

Example 53, Compound 411

IR 2924 2856 2210 1754 1711 1628 1526 1468 1373 1282 1191 1062 1015 816 739
NMR 0.83-0.97(m,6H) 1.14-1.51(m,24H) 1.53-1.72(m,4H) 2.68(t,2H) 4.37(t,2H) 6.91-6.98(m,1H) 7.18-7.28(m,1H) 7.63(dd,1H) 7.33-7.98(m,2H) 8.08-8.12(m,2H) 8.57(s,1H)
$^{19}$F-NMR -59.6(dd,1F) -67.3(dd,1F)

Example 54, Compound 511

IR 2926 2856 2214 1713 1622 1460 1282 1220 1151 1015 955 888 810 777

NMR 0.83-0.96(m,6H) 1.18-1.52(m,24H) 1.64-1.82(m,4H) 2.76(t,2H) 4.34(t,2H) 7.30-7.40(m,2H) 7.53-7.62(m,2H) 7.65-7.72(m,1H) 7.76(d,2H) 8.07(s,1H)

$^{19}$F-NMR -57.9(dd,1F) -58.8(dd,1F)

Example 55, Compound 714

IR 2920 2854 2232 1603 1462 1278 1193 1006 942 886 818 719

NMR 0.83-0.92(m,6H) 1.21-1.41(m,24H) 1.58-1.75(m,4H) 2.66(t,2H) 2.77(t,2H) 6.88-6.95(m,1H) 7.18-7.25(m,1H) 7.36(dd,1H) 7.52-7.62(m,2H) 7.74(d,2H) 8.04(d,1H)

$^{19}$F-NMR -60.0(dd,1F) -67.6(dd,1F)

Compound 420

IR 2922 2852 2306 1715 1495 1379 1378 1224 1185 1098 893 824 746

NMR 0.87(t,3H) 0.97(t,3H) 1.04(d,3H) 1.08-1.42(m,15H) 1.49-1.62(m,1H) 1.64-1.80(m,2H) 1.83-1.98(m,1H) 2.79 (t,2H) 4.13-4.31(m,2H) 7.15(d,1H) 7.62(dd,1H) 7.75 (dd,1H) 7.82-7.96(m,2H) 8.05-8.11(m,2H) 8.57(s,1H) 8.73(d,1H)

$^{19}$F-NMR -60.0(dd,1F) -67.6(dd,1F)

## Claims

1. A liquid crystal compound, represented by the following formula (1):

   R-Z-A-#-NAP-Z′-R′ (1)

   wherein R represents an alkyl group containing 1 to 18 carbon atoms; R′ represent an alkyl group containing 1 to 21 carbon atoms; NAP represents 2,6-naphthylene group; # represents C≡C; A is a cyclic group selected from the group consisting of Pyr>, <Pyr, FPhF, PhF and FPh; Z is -, O or OCO, and Z′ is O, COO or -; wherein Pyr>, <Pyr, FPhF, PhF, FPh and - represent respectively

   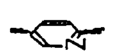 , 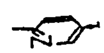 , 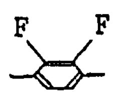 , 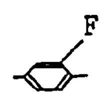 , 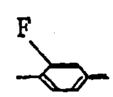

   and a single bond; with the provisos that

   1) if A is Pyr>,
      (a) Z is - and Z′ is O or COO, or
      (b) Z is OCO and Z′ is - or O;
   2) if A is FPhF,
      (a) Z is - or O and Z′ is -, O or COO, or
      (b) Z is OCO and Z′ is - or O;
   3) if A is FPh,
      (a) Z is - and Z′ is O or COO, or
      (a) Z is O and Z′ is -, O or COO, or
      (b) Z is OCO and Z′ is - or O;
   4) if A is <Pyr, Z is - or O and Z′ is COO; and
   5) if A is PhF, Z is O and Z′ is -.

2. A compound according to Claim 1, wherein A is Pyr>, <Pyr or Pym>.

3. A compound according to Claim 1, wherein A is FPhF, FPh or PhF.

4. A compound according to Claim 2, wherein A is Pyr>, and Z and Z′ are preferably as follows:
   (a) Z is - and Z′ is O, or
   (b) Z is -and Z′ is COO.

**5.** A compound according to Claim 3, wherein A is FPhF, and Z and Z′ are preferably as follows:
(a) Z is - and Z′ is O,
(b) Z is O and Z′ is O, or
(c) Z is - and Z′ is O.

**6.** A compound according to Claim 3, wherein A is FPh, and Z and Z′ are preferably as follows:
Z is O and Z′ is -.

**7.** A compound according to Claim 2, wherein A is <Pyr, and Z and Z′ are preferably as follows:
Z is O and Z′ is COO.

**8.** A compound according to any one of Claims 1-7, wherein at least one of R and R′ is an optically active alkyl group, particularly a group represented by the formula:

$$\begin{array}{l} \qquad\qquad\quad CH_3 \\ \qquad\qquad\quad / \\ -(CH_2)_n-{}^{*}CH-R'' \end{array}$$

wherein n is an integer of 0-9 and R″ represents an alkyl group containing 2-10 carbon atoms.

**9.** A liquid crystal composition, containing as components thereof a mixture of a plurality of crystal compounds, said liquid crystal compounds comprising at least one compound according to any one of Claims 1-8, said composition optionally containing a two-tone dyestuff.

**10.** A liquid crystal display element, containing a compound according to any one of Claims 1-8, or a liquid crystal composition according to Claim 9.

European Patent Office

# EUROPEAN SEARCH REPORT

Application Number

EP 92 30 5315

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-3 837 208 (MERCK)<br>* page 2, line 4 - line 33 *<br>* page 4, line 31 - line 32 *<br>* page 5, line 1 *<br>* page 7, line 20 - line 28 *<br>* claims *<br>--- | 1-4,9,10 | C09K19/32<br>C09K19/34<br>C09K19/42<br>C07C43/225<br>C07C43/215<br>C07C69/76<br>C07C69/94<br>C07C43/20<br>C07C43/205<br>C07D213/06<br>C07D213/30<br>C07D213/55<br>C07D213/64<br>C07D213/79 |
| X | EP-A-0 409 634 (SANYO CHEMICAL)<br>* page 4 *<br>* page 9, line 52 *<br>* page 14, line 54 - page 15, line 2 *<br>* table 1(a), compounds no. 119-126 *<br>* example 24 *<br>--- | 1-4 | |
| A | EP-A-0 332 025 (MERCK)<br>* page 2, line 1 - line 25 *<br>* claims 1,3-5 *<br>--- | 1,3,8-10 | |
| A | EP-A-0 385 692 (SANYO CHEMICAL)<br>* page 2, line 26 - page 3, line 5 *<br>* table 1-2, compound no. 75-77 *<br>----- | 1-4,8 | TECHNICAL FIELDS SEARCHED (Int. Cl.5)<br>C09K |

The present search report has been drawn up for all claims

| Place of search | Date of completion of the search | Examiner |
|---|---|---|
| THE HAGUE | 08 SEPTEMBER 1992 | PUETZ C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)